# EUROPEAN PATENT APPLICATION

(11) **EP 3 901 286 A1**
(43) Date of publication of application: **27.10.2021**
(21) Application number: 20382336.4
(22) Date of filing: 24.04.2020
(51) Int. Cl.: C12Q 1/6853

(54) **BIVALENT REVERSE PRIMER**

(71) Applicant: Mirnax Biosens, S.L., 28003 Madrid (ES)
(72) Inventor: CASTÁN GARCÍA, Pablo, 28108 Alcobendas, Madrid (ES); GIL GARCÍA, Ana Isabel, 28108 Alcobendas, Madrid (ES); MADEJÓN SEIZ, Antonio, 28029 Madrid (ES); SAINZ MARTÍNEZ, Enrique, 28108 Alcobendas, Madrid (ES); MONTOYA MIÑANO, Juan José, 28108 Alcobendas, Madrid (ES)
(74) Representative: Hoffmann Eitle

(57) **Abstract**

The present invention refers to a method directed to RT-qPCR reactions, preferably performed in a one or two-step approach combining the reverse transcription and subsequent PCR in a single tube and buffer, using a reverse transcriptase along with a DNA polymerase with mandatory 3' → 5' exonuclease activity, which corrects miss-incorporated nucleotides. In particular, in this invention we present a One-step RT-PCR, preferably qPCR, method with a novel priming strategy that utilizes a novel bivalent reverse primer, wherein this primer is used for both, i) the generation of cDNA and ii) the completion of the subsequent amplification using that same cDNA as template. This bivalent reverse primer also allows end-tagging the amplicon(s) obtained so that they can be used in a variety of applications including, standard sequencing, next generation sequencing (NGS), gene expression analysis, RNAi validation, microarray validation, pathogen detection, genetic testing, and disease research.

## Description

### FIELD OF INVENTION

The present teachings are in the field of molecular and cell biology, specifically in the field of detecting target polynucleotides.

### BACKGROUND OF THE INVENTION

Reverse transcription (RT) and the polymerase chain reaction (PCR) are critical to many molecular biology and related applications, particularly gene expression analysis applications. In these applications, reverse transcription is used to prepare template DNA from an initial RNA sample, e.g. mRNA, which template DNA is then amplified using PCR to produce a sufficient amount of amplified product for the application of interest. The RT and PCR steps of DNA amplification can be carried out as a two-step or one step process. In two step processes, the first step involves synthesis of first strand cDNA with a reverse transcriptase, e.g. MMLV-RT, following by a second PCR step. In certain protocols, these steps are carried out in separate reaction tubes. In these two tube protocols, following reverse transcription of the initial RNA template in the first tube, an aliquot of the resultant product is then placed into the second PCR tube and subjected to PCR amplification.

In a second type of two-step process, both RT and PCR are carried out in the same tube using a compatible RT and PCR buffer. In certain embodiments of single tube protocols, reverse transcription is carried out first, followed by addition of PCR reagents to the reaction tube and subsequent PCR. In an effort to further expedite and simplify RT-PCR procedures, a variety of one step RT-PCR protocols have been developed. However, there is still room for improvement of these methods in a number of areas, including sensitivity, efficiency, and the like. In particular, when these methods are directed to short length nucleotide sequences such as for example miRNAs.

Accordingly, there is continued interest in the development of additional one step RT-PCR protocols, preferably where a highly efficient and sensitive protocol is of particular interest for the production, detection and quantification of short length nucleotide sequences such as for example miRNAs.

### BRIEF DESCRIPTION OF THE FIGURES

**Figure 1****.** A) Fig 1. 1/10 serial dilution curve from synthetic RNA of the miR-127-3p in a concentration range from 6x10⁸ to 6 molecules. B) Slope = -3,654; R2 = 0,993; Eff% = 87,8.
**Figure 2****.** Results obtained for *Staphylococcus aureus* in example 2. In particular, the rDNA sequences that were obtained were compared with sequences in a database made up of sequences from GenBank, EMBL, and the ribosomal database project by using the algorithms provided by each one. For comparison of the rDNA sequences, the FastA program was used. In the table above, the best match and sequence homology are reported according to the original results.
**Figure 3****.** Results obtained for *Escherichia coli* in example 2. The rDNA sequences that were obtained were compared with sequences in a database made up of sequences from GenBank, EMBL, and the ribosomal database project by using the algorithms provided by each one. For comparison of the rDNA sequences, the FastA program was used. In the table, the best match and sequence homology are reported according to the original results.
**Figure 4****.** 1/10 serial dilution curve from synthetic RNA of the miR-144-5p in a concentration range from 6x10⁸ to 6 molecules.
**Figure 5****.** Detection of 1x10⁹ molecules synthetic RNA of the miR-185-5p from triplicates of the same sample.
**Figure 6****.** Detection of 1x10⁹ molecules synthetic RNA of the miR-1246 from triplicates of the same sample.
**Figure 7** illustrates the results for amplification of human RNAse-P template from a clinical blood sample detected using the UNIVERSAL probe annealing with the tagged sequence attached to the forward primer according to the present invention.
**Figure 8** shows comparative results for amplification of human RNAse-P template from a clinical blood sample detected using the standard hRNase P Probe (which anneals with the central sequence of the amplicon generated through the PCR reaction -in green-) vs the UNIVERSAL probe reaction (which anneals with the tagged sequence attached to the Fw primer -in blue.
**Figure 9**. This figure shows comparative results of PCR (light blue) vs ONE STEP RT-qPCR (dark blue) for amplification of human RNAse-P template from a clinical blood sample.
**Figure 10****.** This figure shows fragments that were titrated and pooled to generate the sequencing library introducing the labelled amplicons (Roche Multiplex Identifier, MID) and sequenced in Roche GS Junior device.
**Figure 11****.** This figure shows contigs aligned to reference sequence. Lineage: Eukaryota, Metazoa, Chordata, Craniata, Vertebrata, Euteleostomi, Mammalia, Eutheria, Euarchontoglires, Primates, Haplorrhini, Catarrhini, Hominidae, Homo.

### DESCRIPTION OF THE INVENTION

### DESCRIPTION OF THE INVENTION

### Definitions

As used herein, the term "target polynucleotide" refers to a RNA or DNA polynucleotide sequence that is sought to be detected and/amplified. The target RNA or DNA polynucleotide can be obtained from any source and can comprise any number of different compositional components. For example, the target can be nucleic acid (RNA), transfer RNA, siRNA, miRNA or genomic DNA and can comprise nucleic acid analogs or other nucleic acid mimic. The target can be methylated, non-methylated, or both. Further, it will be appreciated that "target polynucleotide" can refer to the target polynucleotide itself, as well as surrogates thereof, for example amplification products, and native sequences. In some embodiments, the target polynucleotide lacks a poly-A tail. The target polynucleotides of the present teachings can be derived from any number of sources, including without limitation, viruses, prokaryotes, eukaryotes, for example but not limited to plants, fungi, and animals. These sources may include, but are not limited to, whole blood, a tissue biopsy, lymph, bone marrow, amniotic fluid, hair, skin, semen, biowarfare agents, anal secretions, vaginal secretions, perspiration, saliva, buccal swabs, various environmental samples (for example, agricultural, water, and soil), research samples generally, purified samples generally, cultured cells, and lysed cells. It will be appreciated that target polynucleotides can be isolated from samples using any of a variety of procedures known in the art, for example the Applied Biosystems ABI Prism™ 6100 Nucleic Acid PrepStation, and the ABI Prism™ 6700 Automated Nucleic Acid Workstation, Boom et al., U.S. Pat. No. 5,234,809., mirVana RNA isolation kit (Ambion), etc. It will be appreciated that target polynucleotides can be cut or sheared prior to analysis, including the use of such procedures as mechanical force, sonication, restriction endonuclease cleavage, or any method known in the art. In general, the target polynucleotides of the present teachings will be single stranded, though in some embodiments the target polynucleotide can be double stranded, and a single strand can result from denaturation. Preferably the term "target polynucleotide" refers to a miRNA (micro RNA).

As used herein, the term "primer portion" refers to a region of a polynucleotide sequence that can serve directly, or by virtue of its complement, as the template upon which a primer can anneal for any of a variety of primer nucleotide extension reactions known in the art (for example, PCR). It will be appreciated by those of skill in the art that when two primer portions are present on a single polynucleotide, the orientation of the two primer portions is generally different. For example, one PCR primer can directly hybridize to a first primer portion, while the other PCR primer can hybridize to the complement of the primer portion.

As used herein, the term "bivalent reverse primer" refers to a primer that acts as a forward primer for the conservative step of retro-transcription and thus forms a first strand cDNA product from a target RNA polynucleotide. Then a "forward primer" hybridizes with this first cDNA strand product, which is then extended to form a second strand or cDNA product; wherein then the bivalent reverse primer continues the amplification reaction over the second strand product. Preferably, but not limited to, design considerations for the bivalent reverse primer needed for a "one pot/one step" technique presented in this patent to allow the modified/tagged reverse primer for the PCR to act as forward primer for the conservative step of retro-transcription, generating tagged amplicons covering the extent of the RNA region, are herein described as follows:
1. First, the bivalent reverse primer can be of any length but is preferably between 9 to 31, preferably between 16 to 24, nucleotides long and comprises a first sequence of a given base length complementary to one of single strands of the target polynucleotide, preferably a target RNA or miRNA nucleotide, and preferably a second sequence of a given base length provided adjacent to the side of 3'terminus of said first sequence and being non-complementary to any single strands present in the target polynucleotide or to the extension reaction products (amplicons). Generally, the first sequence of the bivalent reverse primer is between 4 and 6 nucleotides in length, and preferably comprises, no more than 71% guanine-cytosine (GC) content over the entire length of said first sequence. Generally, the second sequence is located adjacent to the side of 3'terminus of said first sequence and, as already stated, is not complementary with the extension reaction products; however, the second sequence **allows end-tagging the amplicon(s) obtained so that they can be preferably used in a variety of applications including, standard sanger sequencing or next generation sequencing (NGS), and/or** can hybridize to a detector probe. Generally, the second sequence of the bivalent reverse primer is between 5 and 25 nucleotides long.
2. Secondly and optionally, the bivalent reverse primer preferably has a Melting Temperature (Tm) [defined as the temperature at which one half of the DNA duplex will dissociate to become single stranded] from 48ºC to 72ºC. This range of temperature is calculated, regardless the GC content of the sequence, using the nearest neighbour thermodynamic theory whose formula for primer Tm calculation is:
   Melting Temperature Tm(K)={ΔH/ ΔS + R In(C)}, Or Melting Temperature Tm(oC) = {ΔH/ ΔS + R In(C)} - 273.15 , where:
   i. ΔH (kcal/mole): H is the Enthalpy. Enthalpy is the amount of heat energy possessed by substances. ΔH is the change in Enthalpy. In the above formula the ΔH is obtained by adding up all the di-nucleotide pairs enthalpy values of each nearest neighbour base pair.
   ii. ΔS (kcal/mole): S is the amount of disorder a system exhibits is called entropy. ΔS is change in Entropy. Here it is obtained by adding up all the di-nucleotide pairs entropy values of each nearest neighbour base pair. An additional salt correction is added as the Nearest Neighbour parameters were obtained from DNA melting studies conducted in 1M Na+ buffer and this is the default condition used for all calculations.
   iii. ΔS (salt correction) = ΔS (1M NaCl)+ 0.368 x N x In([Na+]), where:
      1. N is the number of nucleotide pairs in the primer (primer length -1).
      2. [Na+] is salt equivalent in mM.
      3. [Na+] calculation: [Na+] = Monovalent ion concentration +4 x free Mg2+.
3. Thirdly and optionally, the bivalent reverse primer will preferably tolerate a maximum 3' end hairpin with a ΔG of -2 kcal/mol and an internal hairpin with a ΔG of -3 kcal/mol, where ΔG is the Gibbs Free Energy and G is the measure of the amount of work that can be extracted from a process operating at a constant pressure (ΔG = ΔH - TΔS); and
4. Lastly and also optionally, the bivalent reverse primer will preferably need to have a 3'ΔG of -5 kcal/mol and an internal ΔG of -6 kcal/mol to comply with the design while avoiding primer dimer and cross dimer formation. These values are predicted by the algorithms that operate the DNAstar software for design and validation of primers/ sequences/ and modelling of nucleic acids (https://www.dnastar.com/).

As used herein, the term "forward primer" refers to a primer that comprises a first sequence complementary to the first cDNA strand product or to a target DNA polynucleotide, and, optionally, a second sequence or tail portion of a given base length provided adjacent to the side of said first sequence and preferably being non-complementary to any single strands present in the target polynucleotide or in the extension reaction product (amplicons). The first sequence of the forward primer thus hybridizes to a first strand cDNA product or to a target DNA product, such as genomic DNA. Generally, the first sequence of the forward primer is between 4 and 6 nucleotides in length. The tail portion or second sequence is located upstream from the first sequence and **allows end-tagging the amplicon(s) obtained so that they can be preferably used in a variety of applications including, standard sanger sequencing or next generation sequencing (NGS), and/or** can hybridize to a detector probe. Generally, the tail portion of the forward primer is between 5 and 25 nucleotides long. In some embodiments, the tail portion of the forward primer is about 20 nucleotides long.

The term "upstream" as used herein takes on its customary meaning in molecular biology, and refers to the location of a region of a polynucleotide that is on the 5' side of a "downstream" region. Correspondingly, the term "downstream" refers to the location of a region of a polynucleotide that is on the 3' side of an "upstream" region.

As used herein, the term "hybridization" refers to the complementary base-pairing interaction of one nucleic acid with another nucleic acid that results in formation of a duplex, triplex, or other higher-ordered structure, and is used herein interchangeably with "annealing." Typically, the primary interaction is base specific, e.g., A/T and G/C, by Watson/Crick and Hoogsteen-type hydrogen bonding. Base-stacking and hydrophobic interactions can also contribute to duplex stability. Conditions for hybridizing detector probes and primers to complementary and substantially complementary target sequences are well known, e.g., as described in Nucleic Acid Hybridization, A Practical Approach, B. Hames and S. Higgins, eds., IRL Press, Washington, D.C. (1985) and J. Wetmur and N. Davidson, Mol. Biol. 31:349 et seq. (1968). In general, whether such annealing takes place is influenced by, among other things, the length of the polynucleotides and the complementary, the pH, the temperature, the presence of mono- and divalent cations, the proportion of G and C nucleotides in the hybridizing region, the viscosity of the medium, and the presence of denaturants. Such variables influence the time required for hybridization. Thus, the preferred annealing conditions will depend upon the particular application. Such conditions, however, can be routinely determined by the person of ordinary skill in the art without undue experimentation. It will be appreciated that complementarity need not be perfect; there can be a small number of base pair mismatches that will minimally interfere with hybridization between the target sequence and the single stranded nucleic acids of the present teachings. However, if the number of base pair mismatches is so great that no hybridization can occur under minimally stringent conditions then the sequence is generally not a complementary target sequence. Thus, complementarity herein is meant that the probes or primers are sufficiently complementary to the target sequence to hybridize under the selected reaction conditions to achieve the ends of the present teachings.

As used herein, the term "amplifying" refers to any means by which at least a part of a target polynucleotide, target polynucleotide surrogate, or combinations thereof, is reproduced, typically in a template-dependent manner, including without limitation, a broad range of techniques for amplifying nucleic acid sequences, either linearly or exponentially. Exemplary means for performing an amplifying step include ligase chain reaction (LCR), ligase detection reaction (LDR), ligation followed by Q-replicase amplification, PCR, primer extension, strand displacement amplification (SDA), hyperbranched strand displacement amplification, multiple displacement amplification (MDA), nucleic acid strand-based amplification (NASBA), two-step multiplexed amplifications, rolling circle amplification (RCA) and the like, including multiplex versions or combinations thereof, for example but not limited to, OLA/PCR, PCR/OLA, LDR/PCR, PCR/PCR/LDR, PCR/LDR, LCR/PCR, PCR/LCR (also known as combined chain reaction-CCR), and the like. Descriptions of such techniques can be found in, among other places, Sambrook et al. Molecular Cloning, 3rd Edition,; Ausbel et al.; PCR Primer: A Laboratory Manual, Diffenbach, Ed., Cold Spring Harbor Press (1995); The Electronic Protocol Book, Chang Bioscience (2002), Msuih et al., J. Clin. Micro. 34:501-07 (1996); The Nucleic Acid Protocols Handbook, R. Rapley, ed., Humana Press, Totowa, N.J. (2002); Abramson et al., Curr Opin Biotechnol. 1993 February;4(1):41-7, U.S. Pat. No. 6,027,998; U.S. Pat. No. 6,605,451, Barany et al., PCT Publication No. WO 97/31256; Wenz et al., PCT Publication No. WO 01/92579; Day et al., Genomics, 29(1): 152-162 (1995), Ehrlich et al., Science 252:1643-50 (1991); Innis et al., PCR Protocols: A Guide to Methods and Applications, Academic Press (1990); Favis et al., Nature Biotechnology 18:561-64 (2000); and Rabenau et al., Infection 28:97-102 (2000); Belgrader, Barany, and Lubin, Development of a Multiplex Ligation Detection Reaction DNA Typing Assay, Sixth International Symposium on Human Identification, 1995 (available on the world wide web at: promega.com/geneticidproc/ussymp6proc/blegrad.html); LCR Kit Instruction Manual, Cat. #200520, Rev. #050002, Stratagene, 2002; Barany, Proc. Natl. Acad. Sci. USA 88:188-93 (1991); Bi and Sambrook, Nucl. Acids Res. 25:2924-2951 (1997); Zirvi et al., Nucl. Acid Res. 27:e40i-viii (1999); Dean et al., Proc Natl Acad Sci USA 99:5261-66 (2002); Barany and Gelfand, Gene 109:1-11 (1991); Walker et al., Nucl. Acid Res. 20:1691-96 (1992); Polstra et al., BMC Inf. Dis. 2:18-(2002); Lage et al., Genome Res. 2003 February;13(2):294-307, and Landegren et al., Science 241:1077-80 (1988), Demidov, V., Expert Rev Mol Diagn. 2002 November;2(6):542-8., Cook et al., J Microbiol Methods. 2003 May;53(2):165-74, Schweitzer et al., Curr Opin Biotechnol. 2001 February;12(1):21-7, U.S. Patent 5,830,711, U.S. Pat. No. 6,027,889, U.S. Pat. No. 5,686,243, Published P.C.T. Application WO0056927A3, and Published P.C.T. Application WO9803673A1. In some embodiments, newly-formed nucleic acid duplexes are not initially denatured, but are used in their double-stranded form in one or more subsequent steps. An extension reaction is an amplifying technique that comprises elongating a linker probe that is annealed to a template in the 5' to 3' direction using an amplifying means such as a polymerase and/or reverse transcriptase. According to some embodiments, with appropriate buffers, salts, pH, temperature, and nucleotide triphosphates, including analogs thereof, i.e., under appropriate conditions, a polymerase incorporates nucleotides complementary to the template strand starting at the 3'-end of an annealed linker probe, to generate a complementary strand. In some embodiments, the polymerase used for extension lacks or substantially lacks 5' exonuclease activity. In some embodiments of the present teachings, unconventional nucleotide bases can be introduced into the amplification reaction products and the products treated by enzymatic (e.g., glycosylases) and/or physical-chemical means in order to render the product incapable of acting as a template for subsequent amplifications. In some embodiments, uracil can be included as a nucleobase in the reaction mixture, thereby allowing for subsequent reactions to decontaminate carryover of previous uracil-containing products by the use of uracil-N-glycosylase (see for example Published P.C.T. Application WO9201814A2). In some embodiments of the present teachings, any of a variety of techniques can be employed prior to amplification in order to facilitate amplification success, as described for example in Radstrom et al., Mol Biotechnol. 2004 February;26(2):13346. In some embodiments, amplification can be achieved in a self-contained integrated approach comprising sample preparation and detection, as described for example in U.S. Pat. Nos. 6,153,425 and 6,649,378. Reversibly modified enzymes, for example but not limited to those described in U.S. Pat. No. 5,773,258, are also within the scope of the disclosed teachings. The present teachings also contemplate various uracil-based decontamination strategies, wherein for example uracil can be incorporated into an amplification reaction, and subsequent carry-over products removed with various glycosylase treatments (see for example U.S. Pat. No. 5,536,649, and U.S. Provisional Application 60/584,682 to Andersen et al.,). Those in the art will understand that any protein with the desired enzymatic activity can be used in the disclosed methods and kits. Descriptions of DNA polymerases, including reverse transcriptases, uracil N-glycosylase, and the like, can be found in, among other places, Twyman, Advanced Molecular Biology, BIOS Scientific Publishers, 1999; Enzyme Resource Guide, rev. 092298, Promega, 1998; Sambrook and Russell; Sambrook et al.; Lehninger; PCR: The Basics; and Ausbel et al.

As used herein, the term "detector probe" refers to a molecule used in an amplification reaction, typically for quantitative or real-time PCR analysis, as well as end-point analysis. Such detector probes are characterized in that they are from 13 to 21 nucleotides in length, and further characterized in that said probes are sufficiently complementary to the cDNA or DNA products to hybridize under the selected reaction conditions, and in that said complementarity, in some preferred embodiments of the present invention, overlaps in one, two or three overlaps in one, two or three nucleotides with the primer portion of the cDNA or DNA product that serves directly, or by virtue of its complement, as the template upon which the reverse primer or the forward primer anneal. In some embodiments, detector probes present in an amplification reaction are suitable for monitoring the amount of amplicon(s) produced as a function of time.

The term "corresponding" as used herein refers to a specific relationship between the elements to which the term refers. Some non-limiting examples of corresponding include: a linker probe can correspond with a target polynucleotide, and vice versa. A forward primer can correspond with a target polynucleotide, and vice versa. A linker probe can correspond with a forward primer for a given target polynucleotide, and vice versa. The 3' target-specific portion of the linker probe can correspond with the 3' region of a target polynucleotide, and vice versa. A detector probe can correspond with a particular region of a target polynucleotide and vice versa. A detector probe can correspond with a particular identifying portion and vice versa. In some cases, the corresponding elements can be complementary. In some cases, the corresponding elements are not complementary to each other, but one element can be complementary to the complement of another element.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, MB, BBC, AAABCCCC, CBBAAA, CABABB, and so forth. The skilled artisan will understand that typically there is no limit on the number of items or terms in any combination, unless otherwise apparent from the context.

As used herein, the term "reaction vessel" generally refers to any container in which a reaction can occur in accordance with the present teachings. In some embodiments, a reaction vessel can be an eppendorf tube, and other containers of the sort in common practice in modern molecular biology laboratories. In some embodiments, a reaction vessel can be a well in microtitre plate, or a spot on a glass slide. For example, a plurality of reaction vessels can reside on the same support. In some embodiments, lab-on-a-chip like devices, available for example from Caliper and Fluidgm, can provide for reaction vessels. In some embodiments, various microfluidic approaches as described in U.S. Provisional Application 60/545,674 to Wenz et al., can be employed. It will be recognized that a variety of reaction vessel are available in the art and within the scope of the present teachings.

As used herein, the term "detection" refers to any of a variety of ways of determining the presence and/or quantity and/or identity of a target polynucleotide.

Quantitative reverse transcription PCR (RT-qPCR) is a modified amplification method used when the starting material is RNA. In the method described in the present invention, the RNA existing in any given sample is first transcribed into complementary DNA (cDNA) by reverse transcriptase from total RNA or messenger RNA (mRNA). The cDNA is then used as the template for the qPCR reaction.

It is finally noted that all of the methods and systems that form part of the present invention, are compatible with any nucleotide extraction system, such as those commercially available (i.e. Arcis, Qiagen etc...)

### DESCRIPTION

The method referred herein describes PCR (qPCR) or RT-qPCR reactions. Preferably RT-qPCR reactions performed in a one or two-step approach combining the reverse transcription and subsequent PCR in a single tube and buffer, using a reverse transcriptase along with a DNA polymerase, preferably with 3' → 5' exonuclease activity and thus with the ability to correct miss-incorporated nucleotides. In particular, in this invention we present a One-step RT-PCR, preferably RT-qPCR, method with a novel priming strategy that utilizes a novel bivalent reverse primer, wherein this primer is used for both, i) the generation of cDNA and ii) the completion of the subsequent amplification using that same cDNA as template. This bivalent reverse primer additionally allows end-tagging the amplicon(s) obtained so that they can be used in a variety of applications including, standard sequencing, next generation sequencing (NGS), gene expression analysis, RNAi validation, microarray validation, pathogen detection, genetic testing, and disease research or can hybridize to a detector probe.

Therefore, a first aspect of the invention refers to a method (hereinafter first method of the invention) for detecting or amplifying a RNA target polynucleotide in a sample, preferably a human biological sample, performed in a one or two-step approach combining the reverse transcription and subsequent polymerase chain reaction in a single tube and buffer, wherein the method comprises:
- carrying-out a retro-transcription reaction by using **a bivalent primer** that acts as a forward primer for the conservative step of retro-transcription of the RNA target nucleotide and forms a first strand cDNA product from the said RNA target nucleotide; and
- carrying-out a polymerase chain reaction by using a forward primer that hybridizes with the first strand cDNA product which is then extended to form a second cDNA strand product; wherein then the bivalent primer then continues the amplification reaction over the second strand product acting as a reverse primer for the polymerase chain reaction;
wherein the bivalent primer comprises a **first sequence** of a given base length complementary to one primer portion of the RNA target nucleotide that serves directly as the template upon which the primer can anneal and to one primer portion of the second strand or cDNA product that serves directly as the template upon which the primer can anneal; and, optionally,a second sequence of a given base length provided adjacent to the side of 3'terminus of said first sequence and which preferably **allows end-tagging the amplicon(s) obtained so that they can be preferably used in a variety of applications including, standard sequencing, next generation sequencing (NGS), gene expression analysis, RNAi validation, microarray validation, pathogen detection, genetic testing, and disease research and/or** can hybridize to a detector probe. **Such** second sequence of a given base length provided adjacent to the side of 3'terminus of said first sequence is non-complementary to any single strands present in the target polynucleotide or to the extension reaction products (amplicons).

In a preferred embodiment of the first aspect of the invention said reverse primer is preferably further characterized in that the second sequence is between 9 and 31, preferably between 16 to 24, nucleotides long, and wherein the first sequence is between 4 and 20, preferably between 4 and 8, more preferably between 4 and 6 nucleotides in length, and preferably comprises no more than 71% guanine-cytosine (GC) content over the entire length of said first sequence. Preferably, the bivalent reverse primer is preferably further characterized in that:
1. Has a Melting Temperature (Tm) [defined as the temperature at which one half of the DNA duplex will dissociate to become single stranded] from 48ºC to 72ºC.
2. Tolerates a maximum 3' end hairpin with a ΔG of -2 kcal/mol and an internal hairpin with a ΔG of -3 kcal/mol, where ΔG is the Gibbs Free Energy and G is the measure of the amount of work that can be extracted from a process operating at a constant pressure (ΔG = ΔH - TΔS), and
3. Has e a 3'ΔG of -5 kcal/mol and an internal ΔG of -6 kcal/mol to comply with this design while avoiding primer dimer and cross dimer formation.

In another preferred embodiment of the first aspect of the invention, but not limited to, design considerations for the forward primer needed for a "one pot/one step" technique presented in this first aspect are herein described as follows. The forward primer has preferably a structure comprising **a first sequence** of a given base length complementary to one primer portion of the first cDNA product that serves directly as the template upon which the primer can anneal; and, optionally, a second sequence of a given base length provided upstream of said first sequence and which preferably allows end-tagging the amplicon(s) obtained so that they can be preferably used in a variety of applications including, standard sequencing, next generation sequencing (NGS), gene expression analysis, RNAi validation, microarray validation, pathogen detection, genetic testing, and disease research and/or can hybridize to a detector probe. Such second sequence of a given base length is non-complementary to any single strands present in the target polynucleotide or to the extension reaction products (amplicons). The forward primer is preferably further characterized in that the second sequence is between 9 and 31, preferably between 16 to 24, nucleotides long, and wherein the first sequence is between 4 and 20, preferably between 4 and 8, more preferably between 4 and 6 nucleotides in length.

In another preferred embodiment of the first aspect of the invention, detection probes are used, these are preferably characterized as defined in the second aspect of the invention or are capable of hybridizing to the second sequence of the forward primer and/or the bivalent reverse primer and thus detecting the reaction product in view of such complementarity.

In another preferred embodiment of the first aspect of the invention, the method further analyses the results of the amplified products, and preferably determines the presence or absence of the RNA target nucleotide in the biological sample by using the second sequence of the forward primer and/or the bivalent reverse primer that allows end-tagging the amplicon(s) obtained so that they can be preferably used in a variety of applications including, standard sequencing (sanger sequencing), or next generation sequencing (NGS). In this sense, in another preferred embodiment of the first aspect of the invention, the first sequence of the Bivalent primer shall anneal to the template RNA strand and provide reverse transcriptase enzymes a starting point for synthesis incorporating the second sequence that may be used for sequencing or pyrosequencing, in particular for any of the following:
1. Anchoring of a third universal primer compatible with Sanger Sequencing.
2. Anchoring of a third universal primer compatible with modified PCR-based Sequencing used in liquid biopsy.
3. Anchoring of a third universal primer compatible with Next Generation Sequencing.

Alternatively, or complementarily to the above embodiment, the first sequence of the forward primer shall anneal to the template cDNA strand and provide polymerase enzymes a starting point for synthesis incorporating the second sequence that may be used for sequencing or pyrosequencing, in particular for any of the following:
1. Anchoring of a third universal primer compatible with Sanger Sequencing.
2. Anchoring of a third universal primer compatible with modified PCR-based Sequencing used in liquid bopsy.
3. Anchoring of a third universal primer compatible with Next Generation Sequencing.

The examples herein provided demonstrate that those bivalent reverse primers of the invention, first anneal 3'-5' within the RNA sequence of the targeted region acting as a forward primer for the conservative retrotrancription, preferably carried-out at a temperature range of 40º to 50ºC. and secondly anneal 5'-3' within the cDNA+DNA sequences as reverse (Rv) primer at the annealing temperature set for the amplification cycles of the PCR, preferably qPCR, reaction.

Regarding the source(s) for amplification of the first aspect of the invention and in compliance with the technology described in this invention, the target RNA polynucleotide can be obtained from any source and can comprise any number of different compositional components. For example, the target can be nucleic acid (RNA), mRNA, total RNA, transfer RNA, siRNA, miRNA and can comprise nucleic acid analogs or other nucleic acid mimic. In this invention all of these sources are feasible since the PCR's reverse primer is a custom made primer that targets a specific RNA sequence to obtain a specific cDNA pool with increased sensitivity. This primer is preferably used in a greater concentration to compensate for a) its double use (to generate the cDNA in the RT-reaction and to amplify it later in the PCR-reaction) and b) the different annealing efficiency, given that the second sequence may cause it to have a similar behaviour to that of the mixture(s) of oligo(dT)s and random primers. In both cases, the first sequence shall anneal to the template RNA strand and provide reverse transcriptase enzymes a starting point for synthesis and preferably for the incorporation of a second sequence (TAG) that will be used for:
1. Anchoring of a third universal primer compatible with Sanger Sequencing.
2. Anchoring of a third universal primer compatible with modified PCR-based Sequencing used in liquid bopsy.
3. Anchoring of a third universal primer compatible with Next Generation Sequencing.
4. Anchoring of a third universal fluorescent probe and/or dye compatible with standard in vitro RT-qPCR detection and in vivo qPCR and/or RT-qPCR detection, RNA-FISH or dot-blot detection.

Alternatively, to the first aspect, the present invention further relates to a method for detecting or amplifying a DNA target polynucleotide in a sample, preferably a human biological sample, wherein the method comprises carrying-out a polymerase chain reaction (preferably a qPCR) by using a forward primer that hybridizes with a first strand product of the DNA target polynucleotide and a reverse primer that hybridizes to the complementary strand to the first DNA strand product;
wherein the reverse primer comprises a first sequence of a given base length complementary to one primer portion of the DNA target polynucleotide that serves directly as the template upon which the primer can anneal; and, optionally, a second sequence of a given base length provided adjacent to the side of 3'terminus of said first sequence and which preferably allows end-tagging the amplicon(s) obtained so that they can be preferably used in a variety of applications including, standard sequencing, next generation sequencing (NGS), gene expression analysis, RNAi validation, microarray validation, pathogen detection, genetic testing, and disease research and/or can hybridize to a detector probe. Such second sequence of a given base length provided adjacent to the side of 3'terminus of said first sequence is non-complementary to any single strands present in the target polynucleotide or to the extension reaction products (amplicons).

In a preferred embodiment of this alternative aspect of the invention, said reverse primer is preferably further characterized in that the second sequence is between 9 and 31, preferably between 16 to 24, nucleotides long, and wherein the first sequence is between 4 and 20, preferably between 4 and 8, more preferably between 4 and 6 nucleotides in length, and preferably comprises no more than 71% guanine-cytosine (GC) content over the entire length of said first sequence. Preferably, the reverse primer is preferably further characterized in that:
1. Has a Melting Temperature (Tm) [defined as the temperature at which one half of the DNA duplex will dissociate to become single stranded] from 48ºC to 72ºC.
2. Tolerates a maximum 3' end hairpin with a ΔG of -2 kcal/mol and an internal hairpin with a ΔG of -3 kcal/mol, where ΔG is the Gibbs Free Energy and G is the measure of the amount of work that can be extracted from a process operating at a constant pressure (ΔG = ΔH - TΔS), and
3. Has e a 3'ΔG of -5 kcal/mol and an internal ΔG of -6 kcal/mol to comply with this design while avoiding primer dimer and cross dimer formation.

In another preferred embodiment of this alternative aspect of the invention, but not limited to, design considerations for the forward primer are herein described as follows. The forward primer has preferably a structure comprising a first sequence of a given base length complementary to a primer portion of the first strand product of the DNA target polynucleotide that serves directly as the template upon which the primer can anneal; and, optionally, a second sequence of a given base length provided upstream of said first sequence and which preferably allows end-tagging the amplicon(s) obtained so that they can be preferably used in a variety of applications including, standard sequencing, next generation sequencing (NGS), gene expression analysis, RNAi validation, microarray validation, pathogen detection, genetic testing, and disease research and/or can hybridize to a detector probe. Such second sequence of a given base length is non-complementary to any single strands present in the target polynucleotide or to the extension reaction products (amplicons). The forward primer is preferably further characterized in that the second sequence is between 9 and 31, preferably between 16 to 24, nucleotides long, and wherein the first sequence is between 4 and 20, preferably between 4 and 8, more preferably between 4 and 6 nucleotides in length.

In another preferred embodiment of this alternative aspect of the invention, detection probes are used, these are preferably characterized as defined in the second aspect of the invention or are capable of hybridizing to the second sequence of the forward primer and/or the reverse primer and thus detecting the reaction product in view of such complementarity.

In another preferred embodiment of this alternative aspect of the invention, the method further analyses the results of the amplified products, and preferably determines the presence or absence of the DNA target nucleotide in the biological sample by using the second sequence of the forward primer and/or the reverse primer that **allows end-tagging the amplicon(s) obtained so that they can be preferably used in a variety of applications including, standard sequencing (sanger sequencing), or next generation sequencing (NGS).** In this sense, in another preferred embodiment of this alternative aspect of the invention, the first sequence of the forward and/or reverse primer/s shall anneal to the template DNA strand and provide polymerase enzymes a starting point for synthesis incorporating the second sequence that may be used for sequencing (such as sanger sequencing) or pyrosequencing, in particular for any of the following:
1. Anchoring of a third universal primer compatible with Sanger Sequencing.
2. Anchoring of a third universal primer compatible with modified PCR-based Sequencing used in liquid bopsy.
3. Anchoring of a third universal primer compatible with Next Generation Sequencing.

On a separate note, the present invention further resolves the problem of the lack of sufficient space in some specific short target RNA sequences (such as short miRNA sequence of between 15-25 nucleotides in length), caused as a result of the simultaneous union of the two primers, necessary in the amplification, and of the probe necessary for the detection. Under standard conditions, considering an average size of the probes and primers of approximately 20 bases, a fragment of at least 60 bases would be necessary to work with a TaqMan system. In order to solve this problem, the present invention, as shown in the examples, does not only increase the size of the target sequence during the amplification process to allow the binding of the primers and the probe to the amplified products; but, in addition, we herein propose carrying out a **non-canonical form of amplification** in which a detector probe of between 13 to 21 nucleotides in length, and further characterized in that said probe is 100% complementary to a portion of the target RNA sequence, and in that said complementarity overlaps in one, two or three nucleotides with the primer portion of the cDNA product that serves directly, or by virtue of its complement, as the template upon which the reverse or forward primer anneals, is used. It is further noted that such **non-canonical form of amplification** comprises the use of the bivalent primer and a DNA polymerase with exonuclease activity.

Therefore, a second aspect of the invention refers to a method for detecting or amplifying a RNA target nucleotide, preferably a miRNA, of between 15 to 25 nucleotides in length, in a sample, preferably a human biological sample, performed in a one or two-step approach combining the reverse transcription and subsequent polymerase chain reaction in a single tube and buffer, wherein the method comprises:
- carrying-out a retro-transcription reaction by using a bivalent primer that acts as a forward primer for the conservative step of retro-transcription of the RNA target nucleotide and forms a first strand cDNA product from the said RNA target nucleotide; and
- carrying-out a polymerase chain reaction by using a forward primer that hybridizes with the first strand cDNA product which is then extended to form a second cDNA strand product; wherein then the bivalent primer then continues the amplification reaction over the second strand product acting as a reverse primer for the polymerase chain reaction;
characterized in that the DNA polymerase used for carrying-out the polymerase chain reaction possesses a 3'→5' exonuclease activity; and characterized in that a detection probe of from 13 to 21 nucleotides in length is used for detecting the amplified products of the PCR reaction, and further characterized in that said probe is sufficiently complementary to the cDNA products to hybridize under the selected reaction conditions, wherein said complementarity overlaps in one, two or three nucleotides with the primer portion of the cDNA product that serves directly, or by virtue of its complement, as the template upon which the bivalent primer or the forward primer anneal.

In a preferred embodiment of the second aspect of the invention, but not limited to, design considerations for the bivalent reverse primer needed for a "one pot/one step" technique presented in this patent to allow the modified/tagged reverse primer for the PCR to act as forward primer for the conservative step of retro-transcription, generating tagged amplicons covering the extent of the RNA region, are herein described as follows. The bivalent reverse primer has preferably a structure comprising **a first sequence** of a given base length complementary to one primer portion of the RNA target nucleotide that serves directly as the template upon which the primer can anneal and to one primer portion of the second strand or cDNA product that serves directly as the template upon which the primer can anneal; and, optionally, a second sequence of a given base length provided adjacent to the side of 3'terminus of said first sequence and which preferably allows end-tagging the amplicon(s) obtained so that they can be preferably used in a variety of applications including, standard sequencing, next generation sequencing (NGS), gene expression analysis, RNAi validation, microarray validation, pathogen detection, genetic testing, and disease research. The bivalent primer is preferably further characterized in that the second sequence is between 9 and 31, preferably between 16 to 24, nucleotides long, and the first sequence is between 4 and 8, preferably between 4 and 6, nucleotides in length, and preferably comprises no more than 71% guanine-cytosine (GC) content over the entire length of said first sequence. Preferably, the bivalent reverse primer is preferably further characterized in that:
1. Has a Melting Temperature (Tm) [defined as the temperature at which one half of the DNA duplex will dissociate to become single stranded] from 48ºC to 72ºC.
2. Tolerates a maximum 3' end hairpin with a ΔG of -2 kcal/mol and an internal hairpin with a ΔG of -3 kcal/mol, where ΔG is the Gibbs Free Energy and G is the measure of the amount of work that can be extracted from a process operating at a constant pressure (ΔG = ΔH - TΔS), and
3. Has e a 3'ΔG of -5 kcal/mol and an internal ΔG of -6 kcal/mol to comply with this design while avoiding primer dimer and cross dimer formation.

In another preferred embodiment of the second aspect of the invention, but not limited to, design considerations for the forward primer needed for the "one pot/one step" technique presented in this second aspect are herein described as follows. The forward primer has preferably a structure comprising **a first sequence** of a given base length complementary to one primer portion of the first cDNA product that serves directly as the template upon which the primer can anneal; and a second sequence of a given base length provided upstream of said first sequence and which preferably allows end-tagging the amplicon(s) obtained so that they can be preferably used in a variety of applications including, standard sequencing, next generation sequencing (NGS), gene expression analysis, RNAi validation, microarray validation, pathogen detection, genetic testing, and disease research. Alternatively such second sequence is non-complementary to any single strands present in the sample; and said forward primer is preferably further characterized in that the second sequence is between 9 and 31, preferably between 16 to 24, nucleotides long, and wherein the first sequence is between 4 and 8, preferably between 4 and 6, nucleotides in length.

In another preferred embodiment of the second aspect of the invention, the detection probes are from about 17 to about 21 nucleotides in length.

In a preferred embodiment of the second aspect of the invention, the method further analyses the results of the amplified products, and preferably determines the presence or absence of the RNA target nucleotide in the biological sample.

In another preferred embodiment of the second aspect of the invention, the first sequence of the reverse primer shall anneal to the template RNA strand and provide reverse transcriptase enzymes a starting point for synthesis incorporating the second sequence that may be used for any of the following consisting of:
1. Anchoring of a third universal primer compatible with Sanger Sequencing.
2. Anchoring of a third universal primer compatible with modified PCR-based Sequencing used in liquid bopsy.
3. Anchoring of a third universal primer compatible with Next Generation Sequencing.

A third aspect of the invention refers to a method for isolating or purifying the amplified products of a RNA target nucleotide, preferably a miRNA, of between 15 to 25 nucleotides in length, in a sample, preferably a human biological sample, performed in a one or two-step approach combining the reverse transcription and subsequent polymerase chain reaction in a single tube and buffer, wherein the method comprises:
- carrying-out a retro-transcription reaction by using a bivalent primer that acts as a forward primer for the conservative step of retro-transcription of the RNA target nucleotide and forms a first strand cDNA product from the said RNA target nucleotide;
- carrying-out a polymerase chain reaction by using a forward primer that hybridizes with the first strand cDNA product which is then extended to form a second cDNA strand product; wherein then the bivalent primer then continues the amplification reaction over the second strand product acting as a reverse primer for the polymerase chain reaction; and
- isolating or purifying the amplified products;
characterized in that the DNA polymerase used for carrying-out the polymerase chain reaction possesses a 3'→5' exonuclease activity; and preferably characterized in that a detection probe of from 13 to 21 nucleotides in length is used for detecting the amplified products of the PCR reaction, and further characterized in that said probe is sufficiently complementary to the cDNA products to hybridize under the selected reaction conditions, wherein said complementarity overlaps in one or two nucleotides with the primer portion of the cDNA product that serves directly, or by virtue of its complement, as the template upon which the bivalent primer or the forward primer anneal.

In a preferred embodiment of the second aspect of the invention, but not limited to, design considerations for the bivalent reverse primer needed for a "one pot/one step" technique presented in this patent to allow the modified/tagged reverse primer for the PCR to act as forward primer for the conservative step of retro-transcription, generating tagged amplicons covering the extent of the RNA region, are herein described as follows. The bivalent reverse primer has preferably a structure comprising **a first sequence** of a given base length complementary to one primer portion of the RNA target nucleotide that serves directly as the template upon which the primer can anneal and to one primer portion of the second strand or cDNA product that serves directly as the template upon which the primer can anneal; and a second sequence of a given base length provided adjacent to the side of 3'terminus of said first sequence and which preferably allows end-tagging the amplicon(s) obtained so that they can be preferably used in a variety of applications including, standard sequencing, next generation sequencing (NGS), gene expression analysis, RNAi validation, microarray validation, pathogen detection, genetic testing, and disease research. Alternatively such second sequence is non-complementary to any single strands present in the sample. In addition, the bivalent primer is preferably further characterized in that the second sequence is between 9 and 31, preferably between 16 to 24, nucleotides long, and wherein the first sequence is between 4 and 8, preferably between 4 and 6, nucleotides in length, and preferably comprises no more than 71% guanine-cytosine (GC) content over the entire length of said first sequence. Preferably, the bivalent reverse primer is preferably further characterized in that:
1. Has a Melting Temperature (Tm) [defined as the temperature at which one half of the DNA duplex will dissociate to become single stranded] from 48ºC to 72ºC.
2. Tolerates a maximum 3' end hairpin with a ΔG of -2 kcal/mol and an internal hairpin with a ΔG of -3 kcal/mol, where ΔG is the Gibbs Free Energy and G is the measure of the amount of work that can be extracted from a process operating at a constant pressure (ΔG = ΔH - TΔS), and
3. Has e a 3'ΔG of -5 kcal/mol and an internal ΔG of -6 kcal/mol to comply with this design while avoiding primer dimer and cross dimer formation.

In another preferred embodiment of the third aspect of the invention, but not limited to, design considerations for the forward primer needed for a "one pot/one step" technique presented in this patent to allow the modified/tagged reverse primer for the PCR to act as forward primer for the conservative step of retro-transcription, generating tagged amplicons covering the extent of the RNA region, are herein described as follows. The forward primer has preferably a structure comprising a **first sequence** of a given base length complementary to one primer portion of the first cDNA product that serves directly as the template upon which the primer can anneal; and a second sequence of a given base length provided upstream of said first sequence and which preferably allows end-tagging the amplicon(s) obtained so that they can be preferably used in a variety of applications including, standard sequencing, next generation sequencing (NGS), gene expression analysis, RNAi validation, microarray validation, pathogen detection, genetic testing, and disease research. Alternatively such second sequence is non-complementary to any single strands present in the sample; and said forward primer is preferably further characterized in that the second sequence is between 9 and 31, preferably between 16 to 24, nucleotides long, and wherein the first sequence is between 4 and 8, preferably between 4 and 6, nucleotides in length.

In another preferred embodiment of the third aspect of the invention, the detection probes are from about 17 to about 21 nucleotides in length.

In another preferred embodiment of the third aspect of the invention, the first sequence of the reverse primer shall anneal to the template RNA strand and provide reverse transcriptase enzymes a starting point for synthesis incorporating the second sequence that may be used for any of the following consisting of:
1. Anchoring of a third universal primer compatible with Sanger Sequencing.
2. Anchoring of a third universal primer compatible with modified PCR-based Sequencing used in liquid bopsy.
3. Anchoring of a third universal primer compatible with Next Generation Sequencing.

A fourth aspect of the invention refers to the amplified products obtained or obtainable by the first or third aspect of the invention.

In another preferred embodiment of the first to third aspects of the invention, the bivalent reverse primer is present at a greater concentration than the forward primer.

A fifth aspect of the invention refers to kits for use in preparing and/or identifying amplified amounts of DNA from a template RNA(s). The subject kits are characterized by at least including a set of PCR primers, wherein at least one of such primers is the bivalent reverse primer as described in the first or second aspect of the invention. Preferably, the kit further comprises a DNA polymerase used for carrying-out the polymerase chain reaction which preferably possesses a 3'→5' exonuclease activity and/or a reverse transcriptase, as well as preferably at least one of dNTPs and a buffer composition (or the dried precursor reagents thereof, either prepared or present in its constituent components, where one or more of the components may be premixed or all of the components may be separate). In many embodiments, the subject kits will include these additional components, i.e. the kits will include the bivalent reverse primer as described in the first or second aspect as well as the polymerase and reverse transcriptase enzymes, which may be present in a composition as described above or separate, as well as dNTPs and a buffer or components thereof.

In a preferred embodiment of the fifth aspect of the invention, the subject kits are characterized by at least including a set of PCR primers, wherein at least one of such primers is the bivalent reverse primer and the forward primer as described in the first or second aspect of the invention as well. Preferably, the kit further comprises a DNA polymerase used for carrying-out the polymerase chain reaction and/or a reverse transcriptase, as well as preferably at least one of dNTPs and a buffer composition (or the dried precursor reagents thereof, either prepared or present in its constituent components, where one or more of the components may be premixed or all of the components may be separate). In many embodiments, the subject kits will include these additional components, i.e. the kits will include the bivalent reverse primer and the forward primer as described in the first or second aspect as well as the polymerase and reverse transcriptase enzymes, which may be present in a composition as described above or separate, as well as dNTPs and a buffer or components thereof.

In a preferred embodiment of the fifth aspect of the invention, the subject kits are characterized by at least including a set of PCR primers, wherein at least one of such primers is the bivalent reverse primer as described in the first or second aspect of the invention as well as the detection probes as described in the second or third aspect of the invention. More preferably, the kits will include the bivalent reverse primer and the detection probes as described in the second or third aspect as well as the polymerase and reverse transcriptase enzymes, which may be present in a composition as described above or separate, as well as dNTPs and a buffer or components thereof.

By dNTPs is meant a mixture of deoxyribonucleoside triphosphates (dNTPs). Usually the kit will comprise four different types of dNTPs corresponding to the four naturally occurring bases, i.e. dATP, dTTP, dCTP and dGTP. The total amount of dNTPs present in the kit ranges, in many embodiments, from about 1.0 to 1000 µM, usually from about 1.0 to 500 µM and more usually from about 1.0 to 100 µM, where the relative amounts of each of the specific types of dNTPs may be the same or different. See e.g. U.S. patent application Ser. No. 08/960,718, the disclosure of which is herein incorporated by reference.

The aqueous PCR buffer medium that is present in the subject kits includes a source of monovalent ions, a source of divalent cations and a buffering agent. Any convenient source of monovalent ions, such as KCI, K-acetate, NH 4-acetate, K-glutamate, NH4CI, ammonium sulfate, and the like may be employed, where the amount of monovalent ion source present in the buffer will typically be present in an amount sufficient to provide for a conductivity in a range from about 500 to 20,000, usually from about 1000 to 10,000, and more usually from about 3,000 to 6,000 micro-ohms. The divalent cation may be magnesium, manganese, zinc and the like, where the cation will typically be magnesium. Any convenient source of magnesium cation may be employed, including MgCI2, Mg-acetate, and the like. The amount of Mg2+ present in the buffer is one that is elevated as compared to that employed in wild type Taq polymerase systems, and is one that is close to the optimum concentration for MMLV-RT, where the Mg2+ concentration may range from 0.5 to 10 mM, but will preferably range from about 2 to 5 mM. Representative buffering agents or salts that may be present in the buffer include Tris, Tricine, HEPES, MOPS and the like, where the amount of buffering agent will typically range from about 5 to 150 mM, usually from about 10 to 100 mM, and more usually from about 20 to 50 mM, where in certain preferred embodiments the buffering agent will be present in an amount sufficient to provide a pH ranging from about 6.0 to 9.5. Other agents which may be present in the buffer medium include chelating agents, such as EDTA, EGTA and the like and non-ionic detergents, such as Tween 20, Triton X100, NP40, and the like. As mentioned above, the aqueous buffer medium may be present in the subject kits as a fluid or frozen aqueous composition, as dried buffer precursors that may be separate or combined, e.g. as a freeze dried composition.

The subject kits may further include a number of optional components. Optional ingredients that may be present include: a thermostabilizing agent; a glycine based osmolyte, one or more nucleic acids, e.g. oligonucleotides, an RNase inhibitor, and the like. Each of these additional optional components is now described in greater detail.

The first optional component mentioned above is a thermostabilizing agent. The thermostabilizing agent should decrease the rate of denaturation of the reverse transcriptase to allow cDNA synthesis at elevated temperatures, where representative agents include: sugars, e.g. trehaloses, sucrose, raffinose, etc.; polymerase, e.g. PEG, Dextran, polysaccharides, etc.; and the like, where in many embodiments, trehalose is preferred. When included in the subject kits, the amount of thermostabilizing agent will typically range from about 0.9 to 15 mmol, usually from about 0.9 to 3.0 mmol and more usually from about 1.5 to 3.0 mmol.

Another optional component mentioned above is the glycine based osmolyte. Glycine-based osmolytes suitable for use in the present invention include trimethylglycine (BETAINE™), glycine, sarcosine and dimethylglycine. Glycine based osmolytes and their use in amplification reactions are further described in U.S. Pat. No. 5,545,539, the disclosure of which is herein incorporated by reference.

The kits may further include an RNase inhibitor. Suitable RNase inhibitors of interest include: human placental RNase inhibitor, recombinant RNase inhibitor, etc., where recombinant RNase inhibitor is of particular interest in many embodiments.

The kits may further include one or more nucleic acids, where the nucleic acids will generally be oligonucleotides that find use in the reverse transcription or amplification reactions, described in greater detail below. As such, nucleic acids that may be present include oligodTs, random primers and PCR primers. When present, the length of the dT primer will typically range from 12 to 30 nts. In certain embodiments, the oligo dT primer may be further modified to include an arbitrary anchor sequence, where the arbitrary anchor sequence or portion of the primer will typically range from 15 to 25 nt in length.

Other optional components that may be included in the subject kits include: one or more control sets of total RNA, e.g. mouse total RNA, water, and the like.

The various reagent components of the kits may be present in separated containers, or may all be (or in part be) precombined into a reagent mixture for combination with template DNA.

Finally, in many embodiments of the subject kits, the kits will further include instructions for practicing methods of producing amplified amounts of DNA from a template RNA(s), as described in greater detail below, where these instructions may be present on one or more of: a package insert, the packaging, reagent containers and the like.

The above described enzyme compositions and/or kits find use in methods of producing an amplified amount of DNA from a template RNA(s), i.e. producing one or more amplified amounts of DNA from one or more template RNAs. In particular, the above described enzyme compositions and/or kits find use in the one step RT-PCR reactions of the subject invention, as described in greater detail below.

In the subject one-step RT-PCR reactions, an amplified amount of DNA is produced from one or more, usually a plurality of, RNAs in a single reaction container without the sequential addition of reagents to the reaction container. Specifically, the one step RT-PCR methods of the subject invention include the following steps: (a) preparing a reaction mixture; (b) subjecting the prepared reaction mixture to a first set of reverse transcription reaction conditions; and (c) subjecting the reaction mixture to a second set of PCR conditions. Each of these steps is now described separately in greater detail.

The reaction mixture is prepared by combining at least the following components: (a) a DNA polymerase with exonuclease activity; (b) a reverse transcriptase; (c) one or more RNA templates; (d) dNTPs; (e) a quantity of reaction buffer; (f) the bivalent reverse transcription primer; and (g) the forward PCR primers and (h) the detection probes. Other components that may be introduced into the prepared reaction mixture include: (a) a polymerase inhibitor; (b) a thermostabilizing reagent; (c) a glycine based osmolyte; (d) an RNase inhibitor; (e) control RNA and primers; and (f) water. The components are combined in a suitable container, e.g. a thin walled PCR reaction tube.

Following preparation of the reaction mixture, the reaction mixture is first subject to a set of conditions sufficient for reverse transcription of the RNA template present in the reaction mixture to occur, i.e. the reaction mixture is subjected to cDNA synthesis conditions. This first set of conditions is characterized by maintaining the reaction mixture at a substantially constant temperature for a period of time sufficient for cDNA synthesis to occur. The temperature at which the reaction mixture is maintained during this portion of the subject methods generally ranges from about 37 to 55, usually from about 45 to 52 and more usually from about 48 to 50° C. The duration of this step of the subject methods typically ranges from about 15 to 90 min, usually from about 30 to 60 min and more usually from about 50 to 60 min.

The next step of the subject methods is to subject the reaction mixture, which now includes cDNAs which are the result of the reverse transcription of the first step, to PCR conditions for a period of time sufficient for a desired amount of amplified DNA to be produced. The polymerase chain reaction (PCR) is well known in the art, being described in U.S. Pat. Nos. 4,683,202; 4,683,195; 4,800,159; 4,965,188 and 5,512,462, the disclosures of which are herein incorporated by reference. In subjecting the cDNA comprising reaction mixture to PCR conditions during this step of the subject methods, the reaction mixture is subjected to a plurality of reaction cycles, where each reaction cycle comprises: (1) a denaturation step, (2) an annealing step, and (3) a polymerization step. The number of reaction cycles will vary depending on the application being performed, but will usually be at least 15, more usually at least 20 and may be as high as 60 or higher, where the number of different cycles will typically range from about 20 to 40.

The denaturation step comprises heating the reaction mixture to an elevated temperature and maintaining the mixture at the elevated temperature for a period of time sufficient for any double stranded or hybridized nucleic acid present in the reaction mixture to dissociate. For denaturation, the temperature of the reaction mixture will usually be raised to, and maintained at, a temperature ranging from about 85 to 100, usually from about 90 to 98 and more usually from about 93 to 96° C. for a period of time ranging from about 3 to 120 sec, usually from about 5 to 60 sec.

Following denaturation, the reaction mixture will be subjected to conditions sufficient for primer annealing to template DNA present in the mixture. The temperature to which the reaction mixture is lowered to achieve these conditions will usually be chosen to provide optimal efficiency and specificity, and will generally range from about 50 to 75, usually from about 55 to 70° C. Annealing conditions will be maintained for a period of time ranging from about 15 sec to 60 sec.

Following annealing of primer to template DNA or during annealing of primer to template DNA, the reaction mixture will be subjected to conditions sufficient to provide for polymerization of nucleotides to the primer ends in manner such that the primer is extended in a 5' to 3' direction using the DNA to which it is hybridized as a template, i.e. conditions sufficient for enzymatic production of primer extension product. To achieve polymerization conditions, the temperature of the reaction mixture will typically be raised to or maintained at a temperature ranging from about 65 to 75, usually from about 67 to 73° C. and maintained for a period of time ranging from about 15 sec to 20 min, usually from about 30 sec to 5 min.

The above steps of subjecting the reaction mixture to reverse transcription reaction conditions and PCR conditions be performed using an automated device, typically known as a thermal cycler. Thermal cyclers that may be employed for practicing the subject methods are described in U.S. Pat. Nos 5,612,473; 5,602,756; 5,538,871; and 5,475,610, the disclosures of which are herein incorporated by reference.

The subject methods are characterized in that they are extremely efficient. As such, the subject methods can be used to prepare a large amount of amplified DNA from a small amount of template RNA. For example, the subject methods can be used to prepare from about 0.2 to 3.0, usually from about 0.8 to 1.5 µg amplified DNA from an initial amount of 1 ng to 1 µg, usually 100 ng to 500 ng of total RNA template in from about 25 to 40 cycles. The subject methods are also highly sensitive, being able to generate amplified DNA from exceedingly small amounts of template RNA, where by exceedingly small is meant less than about 1 µg, usually less than about 100 ng and more usually less than about 1 ng, where the methods generally require at least about 10 pg template RNA.

The subject one step RT-PCR methods find use in any application where the production of enzymatically produced primer extension product from template RNA is desired, such as the generation of libraries of cDNA from small amounts of mRNA, the generation of gene expression profiles of from or more distinct physiological samples, e.g. as required in gene expression analysis assays, and the like.

The following examples are offered by way of illustration and not by way of limitation.

### EXAMPLES

### Example 1. NON-CANONICAL FORM OF AMPLIFICATION OF SHORT SEQUENCES. One-step RT-qPCR method for detection of short sequences (between 15-25 nucleotides in length) of RNA with TaqMan fluorescent probes.

The present example 1 addresses the problem of the lack of sufficient space in the target sequence (i.e. exemplified herein as a short miRNA sequence of between 15-25 nucleotides in length) caused as a result of the simultaneous union of the two primers, necessary in the amplification, and of the probe necessary for the detection. Under standard conditions, considering an average size of the probes and primers of approximately 20 bases, a fragment of at least 60 bases would be necessary to work with a TaqMan system.

**Solution:** A priori, it appears necessary to increase the size of the target sequence during the amplification process to allow the binding of the primers and the probe to the amplified products, yet, in the present invention, and as illustrated in examples 1 and 2 below, we perform a number of non-canonical forms of amplification. Such non-canonical forms of amplification comprise the use of a detector probe characterized by having 100% complementary to a portion of the target RNA sequence to amplified, wherein said complementarity overlaps in one, two or three nucleotides with the primer portion of the cDNA product that serves directly, or by virtue of its complement, as the template upon which the reverse primer and/or the forward primer anneals, wherein such reverse primer is a bivalent primer (as defined in the present invention). Furthermore, a DNA polymerase with exonuclease activity is used throughout these examples.

### - Materials and methods

A modified Reverse Transcription-Polymerase Chain Reaction (RT-PCR) was used for the target miRNA(s) tested as a highly sensitive and specific method useful for the detection of such short sequences in limiting amounts. The present methodology was carried out as follows:

### 1. In terms of RNA Extraction,

RNA extractions were carried out with the ARCIS Sample Prep Kit (https://arcisbio.com/our-products/arcis-dna-sample-prep-bulk-kit/) for snapshot-extraction and preservation of all nucleic acids according to the manufacturer's instructions. The amount RNA obtained with this procedure (which comes with genomic DNA) can be assessed using different extraction procedures, such as differential display of RTqPCR vs qPCR Ct values, or specific RNA amplification after DNAse I treatment.

### 2. In terms of reverse Transcription-qPCR

RNA obtained was reverse transcribed using the One-Step RT-qPCR Probe Kit, Nzytech and a mix of Forward primer + bivalent reverse primer + MGB probe (please see below) at 5µM for amplification with 5 units of One-Step RT-qPCR Probe Kit (Nzytech).

### PCR Mix/sample used in the RT-qPCRs:

| | |
|---|---|
| Master Mix (One-Step RT-qPCR Probe Kit, Nzytech) | 10 µl |
| AMV-RT (One-Step RT-qPCR Probe Kit, Nzytech) | 1 µl |
| Forward primer 10 µM | 1 µl |
| Reverse primer 40 µM | 1 µl |
| MGB Probe 5 µM | 1 µl |
| H2O (One-Step RT-qPCR Probe Kit, Nzytech) | 5 µl |
| 19 µl PCR Mix + 1 µl RNA | |

### PCR amplification reaction conditions:

| | | | |
|---|---|---|---|
| 25°C | 20 min | | |
| 50°C | 20 min | | |
| | | | |
| 95°C | 10 min | | |
| | | | |
| 95°C | 15 seg | 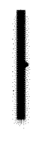 | |
| 25°C | 30 seg | | 5X |
| 60°C | 30 seg | | |
| | | | |
| 95°C | 15 seg | 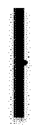 | |
| 40°C | 30 seg | | 45X |
| 60°C | 30 seg | | |

FAM-TAMRA reading within 30 seconds at 60ºC during 45 cycles.

### -Primers and probes used in the hybridization reagents used in the RT-qPCR amplification processes of example 1:

Design of the Forward and Reverse primers:
A.- A 3'-terminal region of 6 nucleotides in length that hybridizes, in the case of the reverse primer with the 6 3'-terminal nucleotides of the target RNA, and in the case of the forward primer with the 6 3'-terminal nucleotides of the sequence complementary to the target RNA.
B.- A sequence of 20 nucleotides in the 5'-terminal position of each primer, of the same sequence in both cases.

Fluorescent probe design:
As illustrated below, each of the probes used in this first example are 100% complementary to a portion of the short miRNA target sequence and said complementarity must overlap in one or maximum three nucleotides with the primer portion of the cDNA product that serves directly, or by virtue of its complement, as the template upon which the primer anneals. Sequence of 14 bases length with the same sequence as the 14 core bases of the RNA substrate. Due to its short length, and to increase the melting temperature, the sequence was synthesized as an MGB(Minor Groove Binding) probe. The 5' end of the probe is labelled with the FAM fluorophore and the 3' end with the MGBEQ quencher.

### Reagents

miR-127-3p:
   F: AATACTACATTAATGTCATTCGGAT
   M: UCGGAUCCGUCUGAGCUUGGCU
   P: ATCCGTCTGAGCTT
   R: AACCGATACTGTAATTACATCATAA
miR-144-5p:
   F: AATACTACATTAATGTCATGGATAT
   M: GGAUAUCAUCAUAUACUGUAAG
   P: ATCATCATATACTG
   R: ACATTCTACTGTAATTACATCATAA
miR-185-5p:
   F: AATACTACATTAATGTCATTGGAGA
   M: UGGAGAGAAAGGCAGUUCCUGA
   P: GAGAAAGGCAGTTC
   R: AGGACTTACTGTAATTACATCATAA
miR-1246:
   F: AATACTACATTAATGTCATAATGG
   M: AAUGGAUUUUUGGAGCAGG
   P: TGGATTTTTGGAGCA
   R: CGTCCTACTGTAATTACATCATAA
   F: Forward Primer.
   M: miRNA.
   P: MGB Probe
   R: Reverse Primer.

### RESULTS

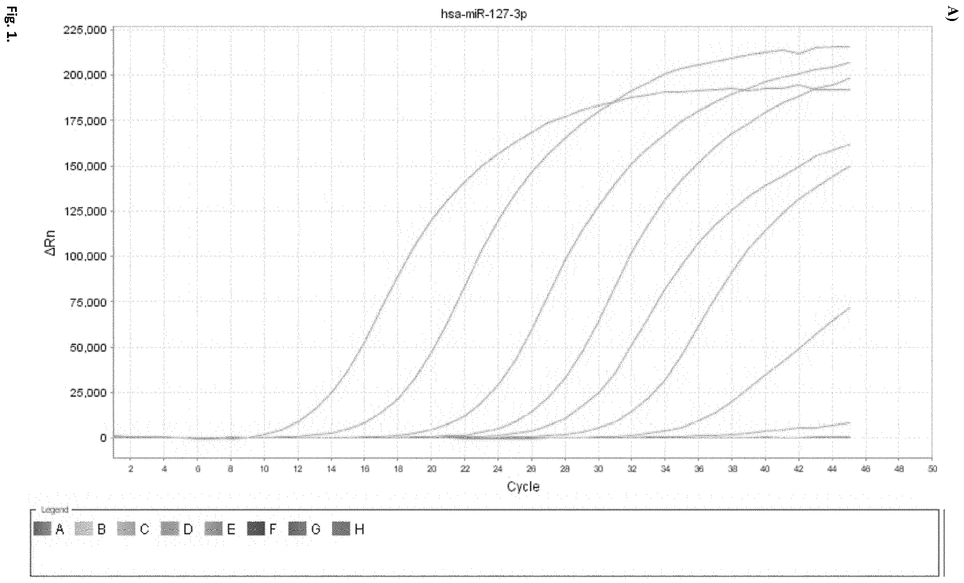

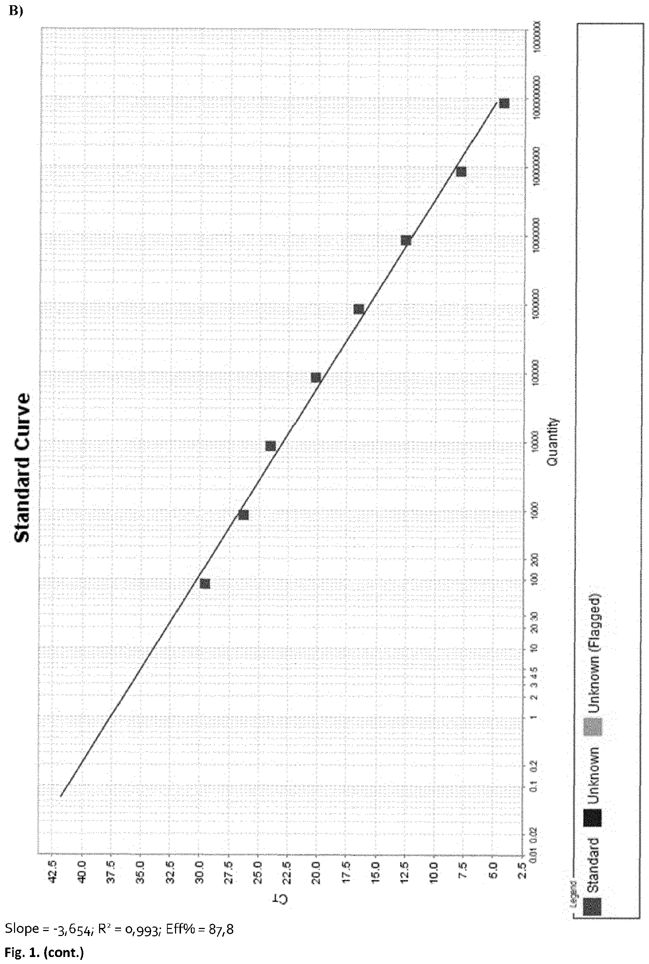

The amplification results for miR-127-3p are shown in Figure 1 and in the table below.

| **Concentration** | **Ct** |
|---|---|
| 6x10⁸ molecules | 4,3 |
| 6x10⁷ molecules | 7,88 |
| 6x10⁶ molecules | 12,61 |
| 6x10⁵ molecules | 1.6,63 |
| 6x10⁴ molecules | 20,24 |
| 6x10³ molecules | 24 |
| 6x10² molecules | 26,34 |
| 6x10¹ molecules | 29,57 |
| 6 molecules | undetermined |

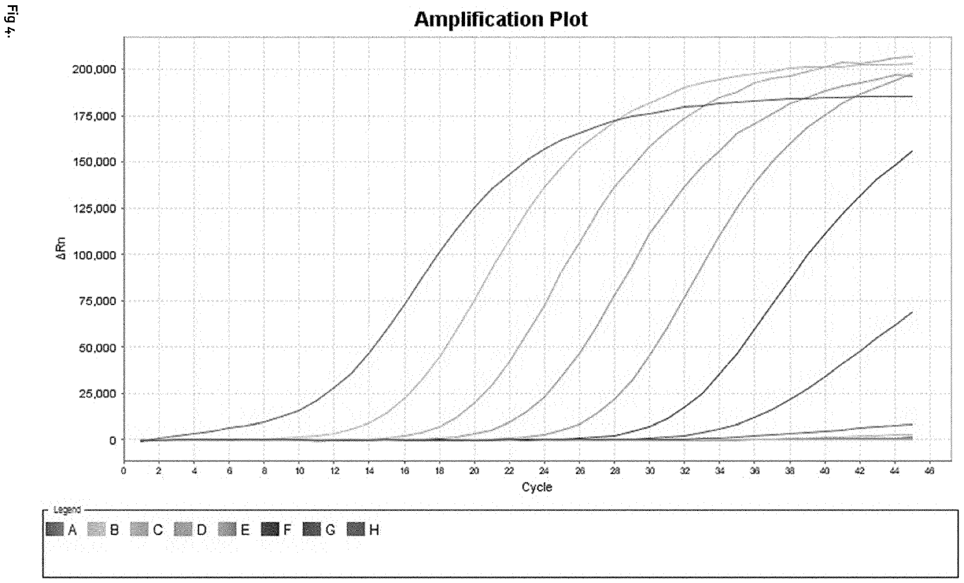

The amplification results for miR-144-5p are shown in figure 4 and in the table below.

| **Concentration** | **Ct** |
|---|---|
| 1x10¹² molecules | undetermined |
| 1x10¹¹ molecules | 7,94 |
| 1x10¹⁰ molecules | 14,52 |
| 1x10⁹ molecules | 17,57 |
| 1x10⁸ molecules | 21,47 |
| 1x10⁷ molecules | 24,88 |
| 1x10⁶ molecules | 28,78 |
| 1x10⁵ molecules | 38,65 |
| 1x10⁴ molecules | undet |
| 1x10³ molecules | undet |
| 1x10² molecules | undet |
| 1x10¹ molecules | undet |

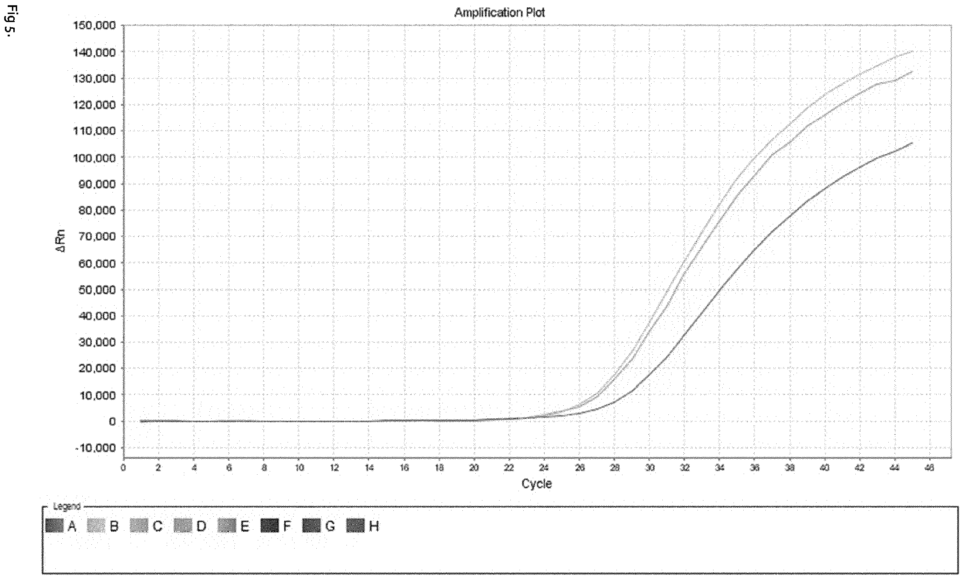

The amplification results for miR-185-5p are shown in figure 5 and in the table below.

| Sample | Ct |
|---|---|
| 1 | 22,66 |
| 2 | 22,98 |
| 3 | 22,27 |

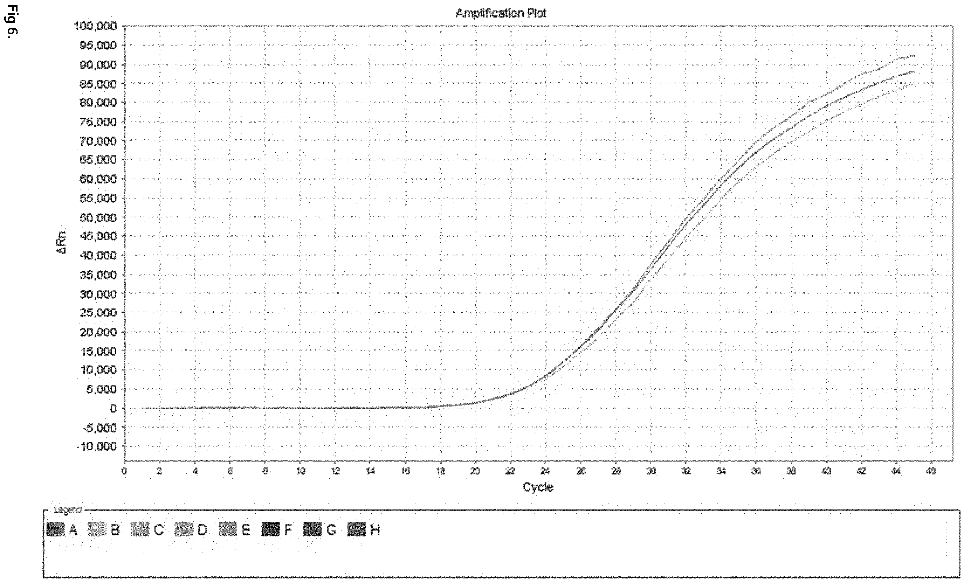

The amplification results for miR-1246 are shown in figure 6 and in the table below.

| Sample | Ct |
|---|---|
| 1 | 16,63 |
| 2 | 16,25 |
| 3 | 17,3 |

### EXAMPLE 2. NON-CANONICAL FORM OF AMPLIFICATION OF LONG RNA TARGET SEQUENCES. Description of RT-qPCR novel solution for universal sequence analysis and application to liquid biopsy.

Quantitative reverse transcription PCR (RT-qPCR) is a modified amplification method used when the starting material is RNA. In the example, the RNA existing in any given test sample is first transcribed into complementary DNA (cDNA) by reverse transcriptase from total RNA or messenger RNA (mRNA). The cDNA is then used as the template for the qPCR reaction.

This example describes a RT-qPCR approach combining the reverse transcription and subsequent PCR in a single tube and buffer, using a reverse transcriptase along with a DNA polymerase. In this example we present a One-step RT-qPCR method (as the one already indicated in the first example) with a novel priming strategy that utilizes a set of modified sequence-specific primers in different concentrations so that one of the primers is used for the generation of cDNA and the completion of the subsequent amplification using that same cDNA as template (the bivalent reverse primer). Those primers also allow end-tagging the amplicon(s) obtained so that they can be used in a variety of applications including, standard sequencing, next generation sequencing (NGS), gene expression analysis, RNAi validation, microarray validation, pathogen detection, genetic testing, and disease research.

Regarding the source(s) for amplification in compliance with the technology described in this example, it is noted that any RNA source such as total RNA or purified mRNA or miRNAs are feasible since the PCR's reverse primer used herein is a custom-made primer that targets any specific RNA sequence to obtain a specific cDNA pool with increased sensitivity. This primer is used in a greater concentration to compensate for a) its double use (to generate the cDNA in the RT-reaction and to amplify it later in the PCR-reaction) and b) the different annealing efficiency, given that the TAG sequence (or second sequence) may cause it to have a similar behaviour to that of the mixture(s) of oligo(dT)s and random primers. In both cases, this tagged primer will anneal to the target iRNA strand and provide reverse transcriptase enzymes a starting point for synthesis incorporating a known sequence (TAG) that can be in turn used for:
1. Anchoring of a third universal primer compatible with Sanger Sequencing.
2. Anchoring of a third universal primer compatible with modified PCR-based Sequencing used in liquid biopsy.
3. Anchoring of a third universal primer compatible with Next Generation Sequencing.

To demonstrate the above concept a blood sample was spiked with a known series of decaying concentrations of bacteria. This was done for a series of representatives of the Gram+ and Gram-groups as explained below.

### - Materials and methods for detection of Staphylococcus aureus subsp. Aureus and Escherichia Coli.

The aim of this experiment was to achieve an accurate phylogenetic classification of two microbes (*Staphylococcus aureus subsp. Aureus* and *Escherichia Coli*) based on rRNA gene (rDNA) sequences. The sequences targeted by the primers used expand regions of up to 400bp that offer a well-defined framework which can be used for microbial identification as the encoded rRNA molecules consist of highly conserved sequences interspersed with regions of more variable sequences. Those highly variable regions are the targeted by this PCR technique so as to make it possible to rationally analyse the sequences covering a desired phylogenetic group of bacteria, whether this group is a certain division, genus, or species.

Extraction of the genetic materials needed for the approach was obtained using the Arcis Pathogen Kit, which is an IVD sample prep system for the release of bacterial nucleic acids from bacteria grown on common microbiological growth substrates. The system was chosen as it has referenced examples of functioning on the following sample types: bacterial cells on agar, liquid broth and standard laboratory buffers such as PBS. Gram Positive and Gram negative bacteria have been used in validation studies including *E.coli, S. aureus* and *K. pneumonia* (https://arcisbio.com/our-products/arcis-dna-pathogen-kit-4/). A specific extraction protocol for blood was followed after the spiking to show compliance of this novel approach in order to guarantee stability of the extracts as per the guideline below:
- Mix 30 ul of spiked blood + 3 ul of 20X ARCIS + 167 ul of water (Extraction Master-Mix)
   1. Mix with vortex.
   2. Incubate for 3 minutes at RT vortexing 3 times for 10 seconds during the process. The sample is then extracted, and the resulting mix is 100% stable at room temperature. Data shows stability vs 50 IU of DNAse-A activity at 37 deg. C for 60 minutes or 10 IU of RNAse activity at 37 deg. C for 60 minutes.
   3. Add 5 ul of the extracted mix to 20 ul of ARCIS washing solution and pipette thoroughly. After mixing with this ratio ¼ the nucleic acids are readily accessible for the application of recombinant DNA technology and procedures, but they no longer remain stable beyond a certain time-threshold (as a rule of thumb we accept 20 minutes for standard gDNA and miRNAs. Specific mRNAs may show different thresholds once washed).
   4. Take 5 ul from the washed extract into the subsequent RTqPCR reaction.

The following three sets of primers were used for a complete retro-transcription of the stabilised extracts under the design of the present invention:

| | |
|---|---|
| µSEQ RTqPCR MiRNAX₁ | FWD 5' - CTGCTGGCACGKAGTTAGCC+TAG₁ |
| | REV 5' - [Protein A bead] ACA CGG YCC AGA CTC+TAG₂ |
| µSEQ RTqPCR MiRNAX₂ | FWD 5' - GAC ARC CAT GCA SCA CCT+TAG₁ |
| | REV 5' - [Protein A bead] GCA ACG CGA AGA+TAG₂ |
| µSEQ RTqPCR MiRNAX₃ | FWD 5' - CTC ACC CGT YCG CCR C+TAG₁ |
| | 5' - [Protein A bead] GAA GAG TTT GAT CAT GG+TAG₂ |

5' chemical modification of the bivalent reverse primer used herein was conjugated to a standard Protein-A bead [Pierce™ Protein A Magnetic Beads (Thermo Scientific™)], although a number of chemical modifications are compatible with this approach such as standard Streptavidin-Biotin conjugation, magnetic beads, haptameric immobilisation... etc. The TAG1 used herein consisted of an 8bp poly-A tail, although virtually any sequence could be designed as suitable TAG as far as the changes in annealing temperature are compensated for the other components of the amplification strategy within the assay. The TAG2 used here consisted of an 8bp poly-T tail, although virtually any sequence could be designed as suitable TAG as far as the changes in annealing temperature are compensated for the other components of the amplification strategy within the assay.

The master-mix for the reaction and the conditions used for the design to undergo RTqPCR was the TaqMan® EXPRESS One-Step SuperScript qRT-PCR Kits (Thermo Fisher), containing HotStar Retro-transcriptase and Taq DNA polymerase plus MgCl2 and premixed dNTPs for a Voltotal reaction: 25 uL (20 master-mix + 5 extracted sample).

The thermocycling conditions applied were:

| | |
|---|---|
| Initial activation | 94 ºC 5 min |
| Retrotranscription | 42 ºC 10 min |
| Initial denaturation | 94 ºC 5 min |
| 40 cycles of: | |
| | |
| Denaturation | 94 ºC 20 sec |
| Annealing | 54 ºC 20 sec |
| Extension | 72 ºC 30 sec |
| | |
| Final extension | 72 ºC 5 min |
| Cooling | 4 ºC ∞ |

### - Materials and methods for the detection of Salmonella enterica subsp. enterica

The aim of this experiment was to achieve the accurate phylogenetic classification of the target microbe (*Salmonella enterica subsp. Enterico*) based on its rRNA gene (rDNA) sequence. The sequences targeted by the primers used expand regions of up to 400bp that offer a well-defined framework which can be used for microbial identification as the encoded rRNA molecules consist of highly conserved sequences interspersed with regions of more variable sequences. Those highly variable regions are the targeted by this PCR technique so as to make it possible to rationally analyse the sequences covering a desired phylogenetic group of bacteria, whether this group is a certain division, genus, or species. The primer sequences are registered in the patent https://patents.google.com/patent/EP2492352A1/en which describes the composition, method and kit for detecting bacteria by means of sequencing the ribosomal RNA using specific areas with taxonomic value.

Briefly, a blood sample was spiked with Salmonella enterica (ATCC 13311) to a final concentration of 10-50 bacteria per microliter of blood. The spiked blood specimen was subjected to the ARCIS extraction protocol as described in the methodology above. Extraction of the genetic materials needed for this approach were thus obtained by using the Arcis Pathogen Kit, which is an IVD sample prep system for the release of bacterial nucleic acids from bacteria grown on common microbiological growth substrates. The system was chosen as it has referenced examples of functioning on the following sample types: bacterial cells on agar, liquid broth and standard laboratory buffers such as PBS. Gram Positive and Gram negative bacteria have been used in validation studies including E.coli, S. aureus and K. pneumonia (https://arcisbio.com/our-products/arcis-dna-pathogen-kit-4/).

The RTqPCR approach was as described in the above methodology (the core sequence of each oligonucleotide belonging to the three primer sets binds to a different position of the bacterial-ribosomal gene resulting fit for sequence analysis) was also followed. For this example, the resulting amplicons were immobilised in a magnetic rack following the protocol defined for the Protein-A bead [Pierce™ Protein A Magnetic Beads (Thermo Scientific™)] placed in 5' of the reverse primer (although a number of chemical modifications are compatible with this approach such as standard Streptavidin-Biotin conjugation, magnetic beads, haptameric immobilisation... etc.).

Immobilised amplicons were immediately treated with denaturing solution following the protocol defined for the use with Qiagen's PyroMark Q96 ID SW 2.5 (https://www.qiagen.com/br/resources/technologies/pyrosequencing-resource center/upgrade-to-pyromark-q96-id-sw/) but substituting the vacuum comb by the magnetic rack. Antiparallel strands were removed as per that protocol and single stranded tagged amplicons were dispensed back to the device to comply with the mechanical part of the pyrosequencing procedure. SQA mode was used using the pattern x20(ACTG), setting the threshold for the initial matching of the 8bp-poly-T TAG2.

### RESULTS

All products were provided to the sequencing services of Instituto de Genética Medica y Molecular (INGEMM), Hospital Universitario La Paz (https://segcd.org/centros/instituto-de-genetica-medica-y-molecular-ingemm-hospital-universitario-la-paz/) to be sequenced using TAG1 and TAG2 as universal sequencing primers in 6 different Sanger sequencing reactions whose results blasted directly allowing identification of the targets initially spiked in blood as per the sequence contigs aligned below:
Dilution 1/100000>*Staphylococcus aureus subsp. aureus*
   (T); ATCC 12600. 
CACCCCAATCATTTGTCCCACCTTCGACGGCTAGCTCCTAAAAGGTTACTCCACCGGCTTCGGGTGTTAC
AAACTCTCGTGGTGTGACGGGCGGTGTGTACAAGACCCGGGAACGTATTCACCGTAGCATGCTGATCT
ACGATTACTAGCGATTCCAGCTTCATGTAGTCGAGTTGCAGACTACAATCCGAACTGAGAACAACTTTA
TGGGATTTGCTTGACCTCGCGGTTTCGCTGCCCTTTGTATTGTCCATTGTAGCACGTGTGTAGCCCAAAT
CATAAGGGGCATGATGATTTGACGTCATCCCCACCTTCCTCCGGTTTGTCACCGGCAGTCAACTTAGAG
TGCCCAACTTAATGATGGCAACTAAGCTTAAGGGTTGCGCTCGTTGCGGGACTTAACCCAACATCTCAC
GACACGAGCTGACGACAACCATGCACCACCTGTCACTTTGTCCCCCGAAGGGGAAGGCTCTATCTCTAG
AGTTGTCAAAGGATGTCAAGATTTGGTAAGGTTCTTCGCGTTGCTTCGAATTAAACCACATGCTCCACC
GCTTGTGCGGGTCCCCGTCAATTCCTTTGAGTTTCAACCTTGCGGTCGTACTCCCCAGGCGGAGTGCTTA
ATGCGTTAGCTGCAGCACTAAGGGGCGGAAACCCCCTAACACTTAGCACTCATCGTTTACGGCGTGGA
CTACCAGGGTATCTAATCCTGTTTGATCCCCACGCTTTCGCACATCAGCGTCAGTTACAGACCAGAAAGT
CGCCTTCGCCACTGGTGTTCCTCCATATCTCTGCGCATTTCACCGCTACACATGGAATTCCACTTTCCTCT
 
TCTGCACTCAAGTTTTCCAGTTTCCAATGACCCTCCACGGTTGAGCCGTGGGCTTTCACATCAGACTTAA
AAAACCGCCTACGCGCGCTTTACGCCCAATAATTCCGGATAACGCTTGCCACCTACGTATTACCGCGGC
TGCTGGCACGTAGTTAGCCGTGGCTTTCTGATTAGGTACCGTCAAGATGTGCACAGTTACTTACACATA
TGTTCTTCCCTAATAACAGAGTTTTACGATCCGAAGACCTTCATCACTCACGCGGCGTTGCTCCGTCAGG
CTTTCGCCCATTGCGGAAGATTCCCTACTGCTGCCTCCCGTAGGAGTCTGGACCGTGTCTCAGTTCCAGT
GTGGCCGATCACCCTCTCAGGTCGGCTATGCATCGTTGCCTTGGTAAGCCGTTACCTTACCAACTAGCTA
ATGCAGCGCG
GATCCATCTATAAGTGACAGCAAGACCGTCTTTCACTTTTGAACCATGCGGTTCAAAATATTATCCGGTA
TTAGCTCCGGTTTCCCGAAGTTATCCCAGTCTTATAGGTAGGTTATCCACGTGTTACTCACCCGTCCGCC
GCTAACATCAGAGAAGCAAGCTTCTCGTCCGTTCGCTCGACTTGCATGTATTAGGCACGCCGCCAGCGT
TCATCCT
Dilution 1/100000>*Escherichia coli*
   (T); ATCC 11775T. 
CGCCCTCCCGAAGTTAAGCTACCTACTTCTTTTGCAACCCACTCCCATGGTGTGACGGGC
GGTGTGTACAAGGCCCGGGAACGTATTCACCGTGGCATTCTGATCCACGATTACTAGCGA
TTCCGACTTCATGGAGTCGAGTTGCAGACTCCAATCCGGACTACGACGCACTTTATGAGG
TCCGCTTGCTCTCGCGAGGTCGCTTCTCTTTGTATGCGCCATTGTAGCACGTGTGTAGCC
CTGGTCGTAAGGGCCATGATGACTTGACGTCATCCCCACCTTCCTCCAGTTTATCACTGG
CAGTCTCCTTTGAGTTCCCGGCCGGACCGCTGGCAACAAAGGATAAGGGTTGCGCTCGTT
GCGGGACTTAACCCAACATTTCACAACACGAGCTGACGACAGCCATGCAGCACCTGTCTC
ACGGTTCCCGAAGGCACATTCTCATCTCTGAAAACTTCCGTGGATGTCAAGACCAGGTAA
GGTTCTTCGCGTTGCATCGAATTAAACCACATGCTCCACCGCTTGTGCGGCCCCCGTCAA
TTCATTTGAGTTTTAACCTTGCGGCCGTACTCCCCAGGCGGTCGACTTAACGCGTTANNT
CCGGAAGCCACGCCTCAAGGGCACAACCTCCAAGTCGACATCGTTTACGGCGTGGACTAC
CAGGGTATCTAATCCTGTTTGCTCCCCACGCTTTCGCACCTGAGCGTCAGTCTTCGTCCA
GGGGGCCGCCTTCGCCACCGGTATTCCTCCAGATCTCTACGCATTTCACCGCTACACCTG
GAATTCTACCCCCCTCTACGAGACTCAAGCTTGCCAGTATCAGATGCAGTTCCCAGGTTG
AGCCCGGGGATTTCACATCTGACTTAACAAACCGCCTGCGTGCGCTTTACGCCCAGTAAT
TCCGATTAACGCTTGCACCCTCCGTATTACCGCGGCTGCTGGCACGGAGTTAGCCGGTGC
TTCTTCTGCGGGTAACGTCAATGAGCAAAGGTATTAACTTTACTCCCTTCCTCCCCGCTG
AAAGTACTTTACAACCCGAAGGCCTTCTTCATACACGCNGCATGGCTGCATCAGGCTTGC
GCCCATTGTGCAATATTCCCCACTGCTGCCTCCCGTAGGAGTCTGGACCGTGTCTCAGTT
CCAGTGTTGCTGGTCATCCTCTCAGACCAGCTAGGGATCGTCGCCTAGGTGAGCCGTTAC
CCCACCTACTAGCTAATCCCATCTGGGCACATCCGATGGCAAGAGGCCCTAAGGTCCCCC
TCTTTGTGCTTGCGACGTTATGCGGTATTAGCTACCGTTTCCAGTAGTTATCCCCCTCCA
TCAGGCAGTTTCCCAGACATTACTCACCCGTCCGCCACTCGTCAGCAAAGCAGCAAGCTG
CTTCCTGTTACCGTTCGACTTGCATGTGTTAGGCCTGCCGCCAGCGTTCAATCTGAGCCA
TGATCAAACT
Search mode> Full search
   Mean identity score> 100%
   Search engine> PyroMark Q96 ID
   Reference database> HULPII
   >Salmonella enterica subsp. enterica (T); ATCC 13311.

TAAGGAGGTGATCCAACCGCAGGTTNCCCTACGGTTACCTTGTTACGACTTCACCCCAGTCATGAATCA
CAAAGTGGTAAGCGCCCTCCCGAAGGTTAAGCTACCTACTTCTTTTGCAACCCACTCCCATGGTGTGAC
GGGCGGTGTGTACAAGGCCCGGGAACGTATTCACCGTGGCATTCTGATCCACGATTACTAGCGATTCC
GACTTCATGGAGTCGAGTTGCAGACTCCAATCCGGACTACGACGCACTTTATGAGGTCCGCTTGCTCTC
GCGAGGTCGCTTCTCTTTGTATGCGCCATTGTAGCACGTGTGTAGCCCTGGTCGTAAGGGCCATGATGA
CTTGACGTCATCCCCACCTTCCTCCAGTTTATCACTGGCAGTCTCCTTTGAGTTCCCGACCTAATCGCTGG
CAACAAAGGATAAGGGTTGCGCTCGTTGCGGGACTTAACCCAACATTTCACAACACGAGCTGACGACA
GCCATGCAGCACCTGTCTCACAGTTCCCGAAGGCACCAATCCATCTCTGGATTCTTCTGTGGATGTCAA
GACCAGGTAAGGTTCTTCGCGTTGCATCGAATTAAACCACATGCTCCACCGCTTGTGCGGCCCCCGTCA
ATTCATTTGAGTTTTAACCTTGCGGCCGTACTCCCCAGGCGGTCTACTTAACGCGTTACGTCCGGAAGCC
ACGCCTCAAGGGCACAACCTCCAAGTAGACATCGTTTACGGCGTGGACTACCAGGGTATCTAATCCTGT
TTGCTCCCCACGCTTTCGCACCTGAGCGTCAGTCTTTGTCCAGGGGGCCGCCTTCGCCACCGGTATTCCT
CCAGATCTCTACGCATTTCACCGCTACACCTGGAATTCTACCCCCCTCTACAAGACTCAAGCCTGCCAGT
TTCGAATGCAGTTCCCAGGTTGAGCCCGGGGATTTCACATCCGACTTGACAGACCGCCTGCGTGCGCTT
TACGCCCAGTAATTCCGATTAACGCTTGCACCCTCCGTATTACCGCGGCTGCTGGCACGGAGTTAGCCG
GTGCTTCTTCTGCGGGTAACGTCAATTGCTGCGGTTATTAACCACAACACCTTCCTCCCCGCTGAAAGTA
CTTTACAACCCGAAGGCCTTCTTCATACACGC

### - Staphylococcus aureus.

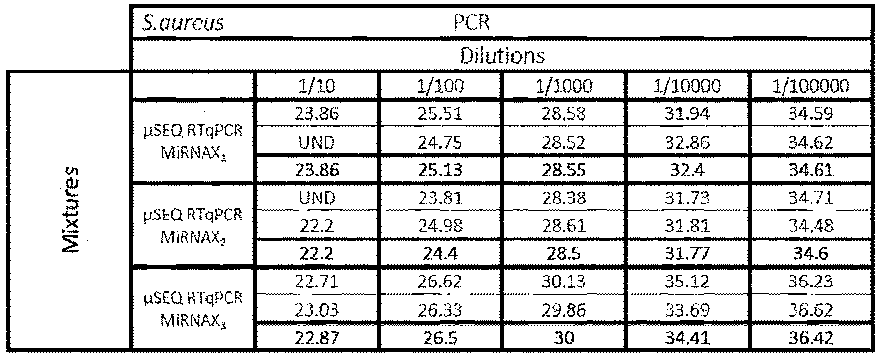

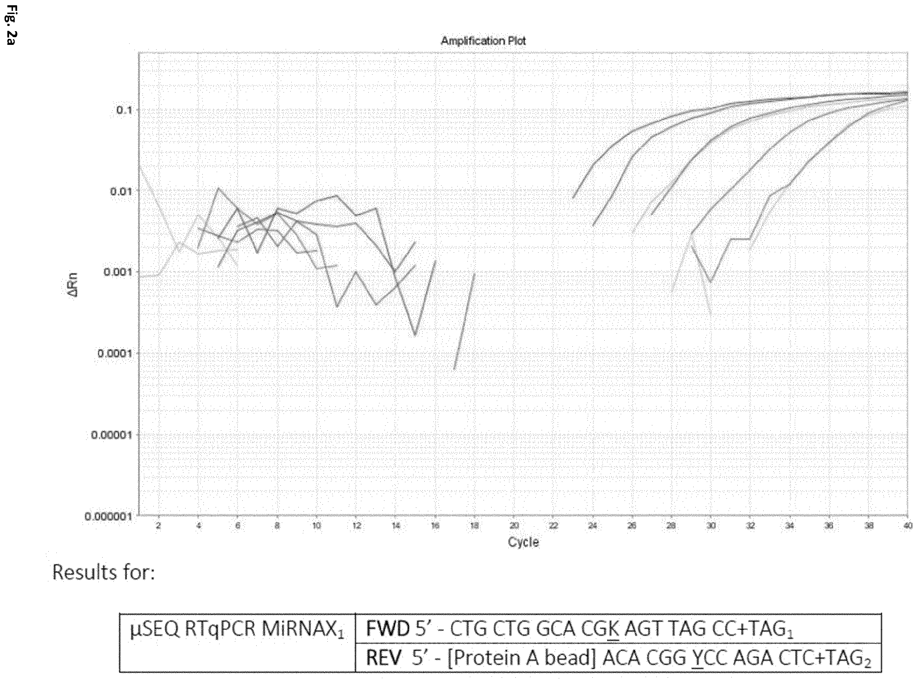

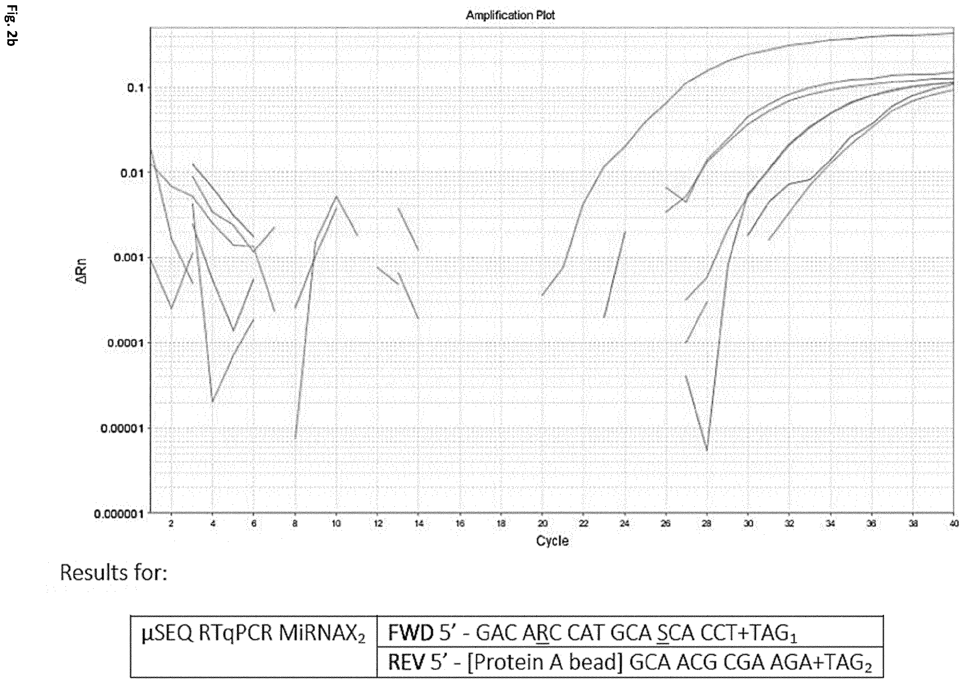

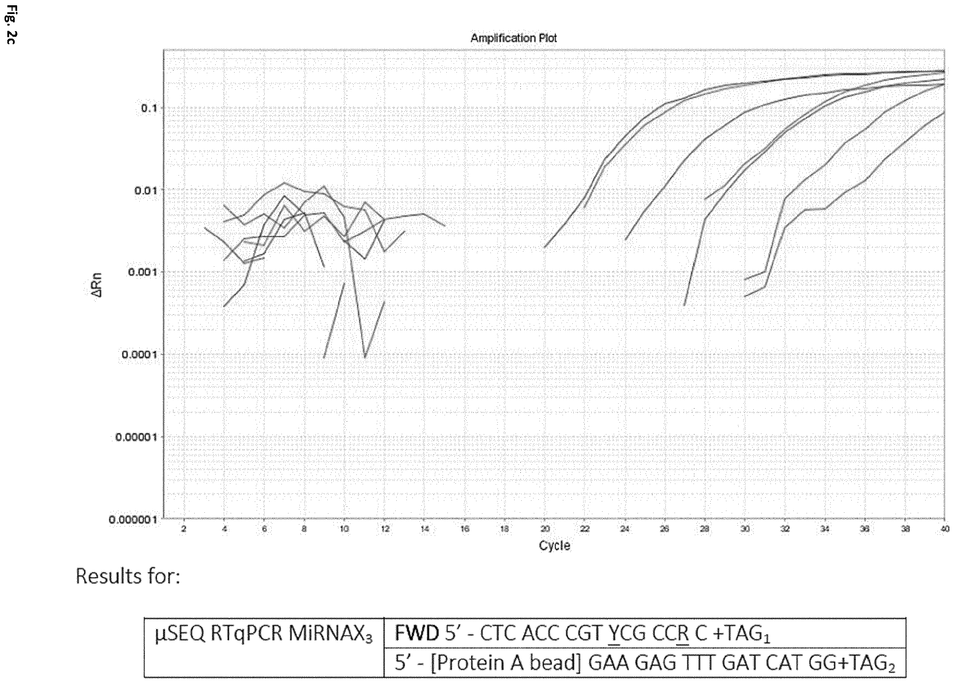

The results are shown in figure 2, in particular, the rDNA sequences that were obtained were compared with sequences in a database made up of sequences from GenBank, EMBL, and the ribosomal database project by using the algorithms provided by each one. For comparison of the rDNA sequences, the FastA program was used. In the table above, the best match and sequence homology are reported according to the original results.

Analytic sensitivity of the assay:
When serial dilutions of the target bacterial cells were used as the template, the analytical sensitivity of the identification predicted on the sequence analysis of the rDNA PCR was about 10 CFU/reaction (accepting a 3Ct delay per log reduction and generation of a negative result over Ct39).

### - Escherichia coli.

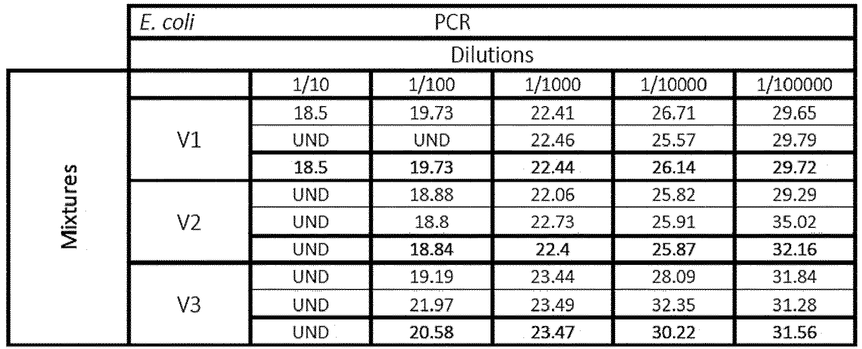

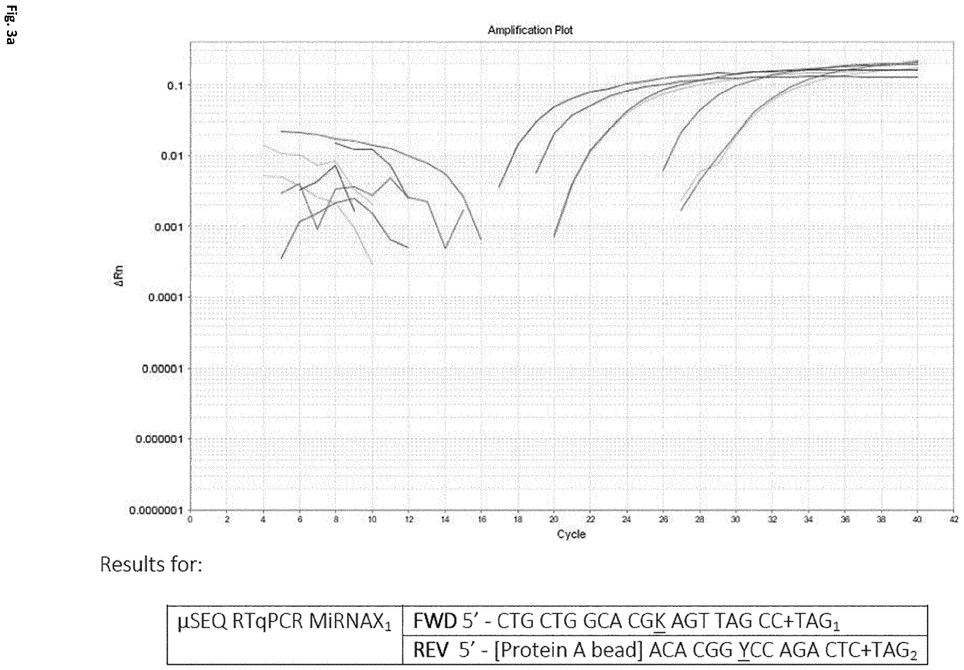

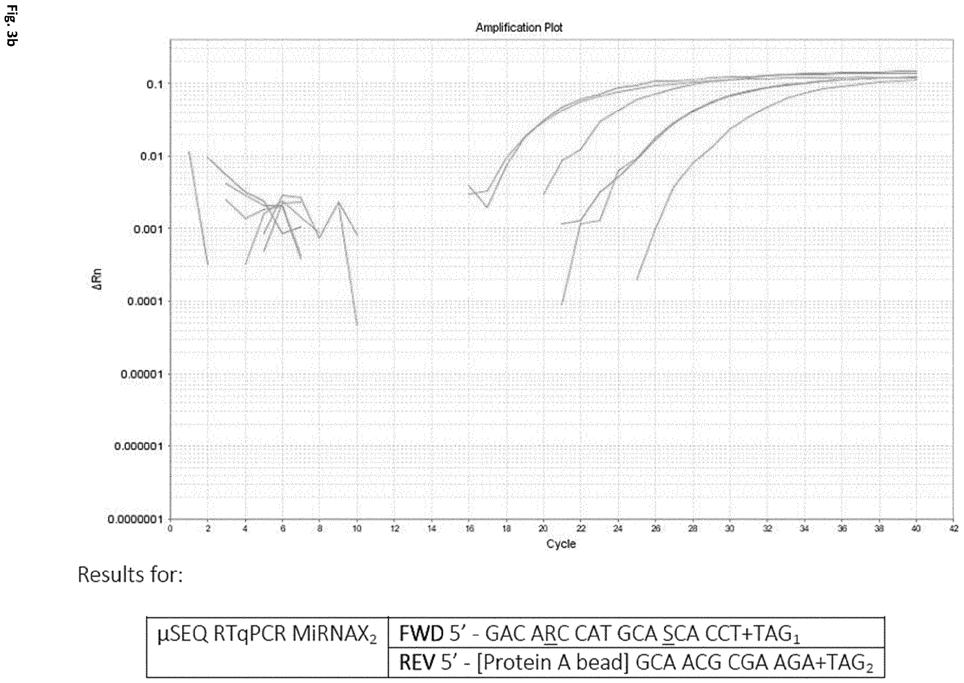

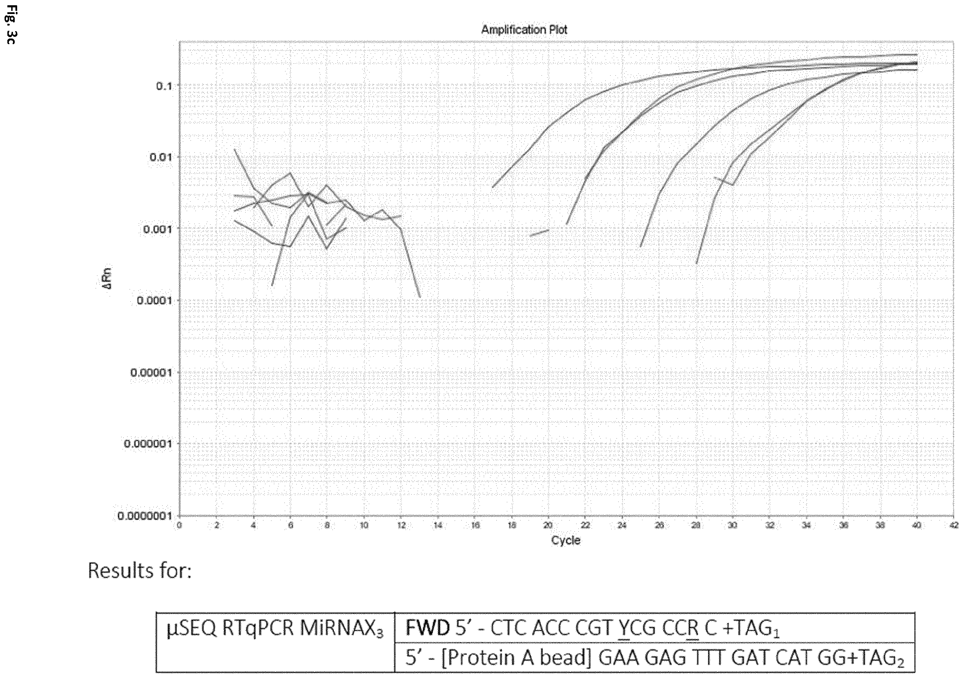

The results are shown in figure 3, wherein the rDNA sequences that were obtained were compared with sequences in a database made up of sequences from GenBank, EMBL, and the ribosomal database project by using the algorithms provided by each one. For comparison of the rDNA sequences, the FastA program was used. In the above table, the best match and sequence homology are reported according to the original results.

Analytic sensitivity of the assay:
When serial dilutions of the target bacterial cells were used as the template, the analytical sensitivity of the identification predicted on the sequence analysis of the rDNA PCR was about 1CFU/reaction (accepting a 3Ct delay per log reduction and generation of a negative result over Ct39).

### - Salmonella enterica subsp. enterica

The reads obtained were directly blasted for homology using a database made up of sequences from GenBank, EMBL and the ribosomal database project. The resulting match allowed identification to the level of species with 99% homology. This score shows compliance of the tagged reverse PCR-primer with Pyrosequencing as example of the modified PCR-based Sequencing methods currently applied to liquid biopsy.

### EXAMPLE 3. DETECTION OF GENOMIC DNA (gDNA)

In this example, detection and identification of the human RNAse-P gene target (gDNA) was performed by using universal tagged probe sequences for universal detection of genome DNA amplicons through qPCR. Amplification was carried-out by using supernatants obtained from the ARCIS blood extraction and preservation protocol using the TaqMan® Universal PCR Master Mix (Applied Biosystems), containing HotStar Taq DNA polymerase, MgCl2 and dNTP's, as indicated below.

The hRNAse-P target was chosen as reference gene for this analysis as it has been shown that human nuclear RNase P is required for the normal and efficient transcription of various small noncoding RNAs, such as tRNA, 5S rRNA, SRP RNA and U6 snRNA genes, which are transcribed by RNA polymerase III, one of three major nuclear RNA polymerases in human cells. This makes it a major control for expression analysis and the target of choice for detection of presence of ghDNA.

Standard hRNAse-P reaction for detection of the gDNA encoding the human RNAse-P enzyme:
- RNase P Primer F: 5'-AGA TTTGGACCTGCGAGCG-3'
- RNase P Primer R: 5'-GAGCGGCTGTCTCCACAAGT-3'
- RNase P Probe: 5'-FAM/VIC-TTCTGACCTGAAGGCTCTGCGCG-BHQ1-3'

In addition, amplification was also carried-out, departing from the same supernatants, by using the present invention's hRNAse-P reaction reagents for the detection of the gDNA encoding the human RNAse-P enzyme:
- RNase P Primer F (TAG sequence in bold): 5'-(GATCGATCGATC)AGA TTTGGACCTGCGAGCG-3'
- RNase P Primer R: 5'-GAGCGGCTGTCTCCACAAGT-3'
- MiRNAX UNIVERAL Probe: 5'-FAM/VIC-GATCGATCGATC-BHQ1-3'

In it noted that for both reactions, the following conditions were used. qPCR reaction master-mix and qPCR parameters for amplification with the TaqMan® Universal PCR Master Mix (Applied Biosystems):

– PCR Master Mix: 12.5 μl
– Primer Fw : 0.5 μl
– Primer Rv: 0.5 μl
– Primer Probe : 0.5 μl
– H2O: 6.0 μl
Mixture volume: 20 μl

Initial denaturation 95 °C 10 min

Denaturation 95 °C 15 sec
Annealing 60 °C 60 sec
45 cycles

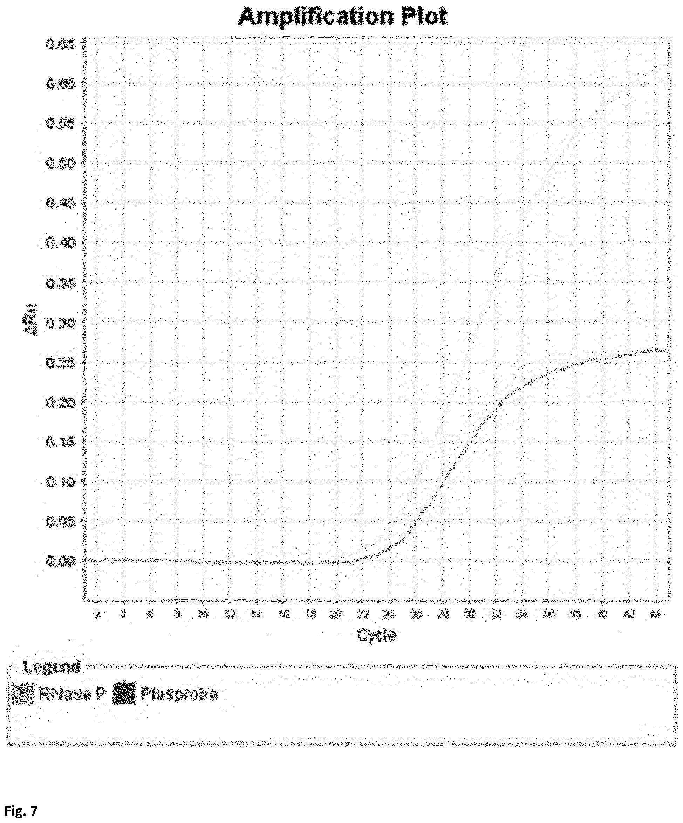

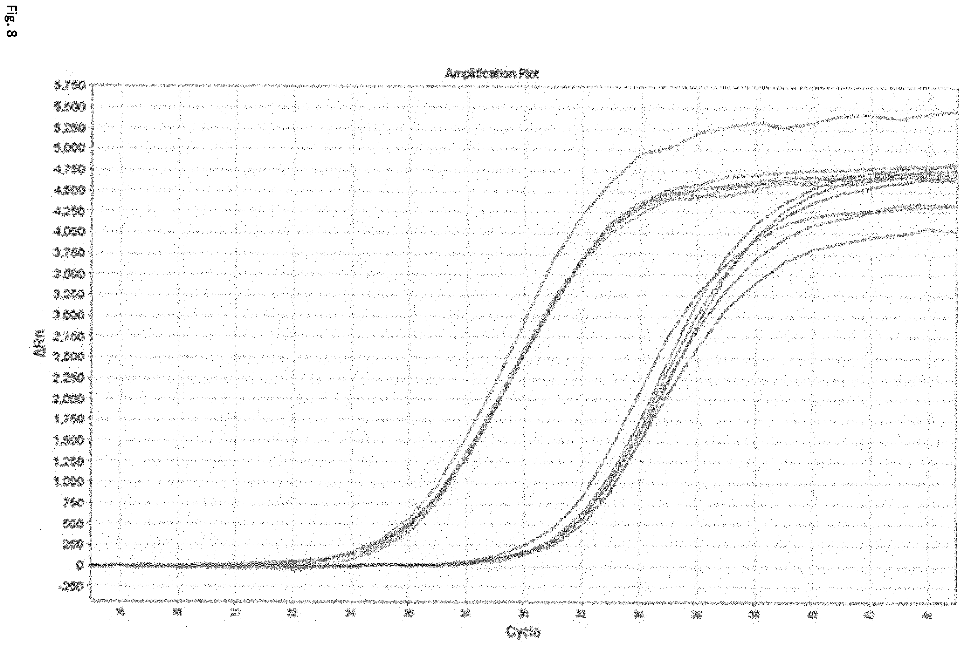

Figure 7 illustrates the results for amplification of human RNAse-P template from a clinical blood sample detected using the UNIVERSAL probe annealing with the tagged sequence attached to the forward primer according to the present invention. Figure 8 shows comparative results for amplification of human RNAse-P template from a clinical blood sample detected using the standard hRNase P Probe (which anneals with the central sequence of the amplicon generated through the PCR reaction -in green-) vs the UNIVERSAL probe reaction (which anneals with the tagged sequence attached to the Fw primer -in blue.

### EXAMPLE 4. DETECTION OF MESSENGER RNA (mRNA)

In this example, detection and identification of human RNAse-P gene target (gDNA) was carried-out by using a reverse primer, in the PCR reaction, which, at the same time, was suitable as a forward primer for the conversion of mRNA to cDNA, which was in turn amplified and detected using a UNIVERSAL probe.

In particular, the PCR amplification reaction indicated herein was carried-out by using supernatants from the ARCIS blood extraction and preservation protocol using the TaqMan® Universal PCR Master Mix (Applied Biosystems), containing HotStarTaq DNA polymerase, MgCl2 and dNTP's. In parallel, a RT-PCR amplification from the same supernatants was performed by using the TaqMan® AgPath Universal RT-qPCR Master Mix (Applied Biosystems).

The hRNAse-P RT-qPCR reaction (ONE STEP RT-qPCR) for detection of the gDNA encoding the human RNAse-P enzyme in accordance with the present invention comprised:
- RNase P Primer F (TAG sequence in italic): 5'-(*GATCGATCGATC*)AGA TTTGGACCTGCGAGCG-3'
- RNase P Primer R (acting here as forward primer for the retro-transcription assay in bold): 5'-**GAGCGGCTGTCTCCACAAGT**-3'
- MiRNAX UNIVERAL Probe: 5'-FAM/VIC-GATCGATCGATC-BHQ1-3'

qPCR reaction master-mix and qPCR parameters for amplification with the TaqMan® Universal PCR Master Mix (Applied Biosystems):

– PCR Master Mix: 12.5 μl
– RnaseP Fw : 0.5 μl
– RnaseP Rv: 0.5 μl
– RnaseP Probe : 0.5 μl
– H₂O: 6.0 μl

Mixture volume: 20 μl

RT-qPCR reaction master-mix and qPCR parameters for amplification with the TaqMan® Universal PCR Master Mix (Applied Biosystems):

– RT-qPCR Master Mix: 12.5 μl
– RnaseP Fw : 0.5 μl
– RnaseP Rv: 0.5 μl
– RnaseP Probe : 0.5 μl
– H2O: 6.0 μl
Mixture volume: 20 μl

RT-qPCR step | 50 °C 2 min
Initial denaturation | 95 °C 10 min
Denaturation | 95 °C 15 sec | 45 cycles
Annealing | 60 °C 60 sec

The same conditions as indicated above were used for the ONE STEP RT-qPCR for amplification of human RNAse-P template.

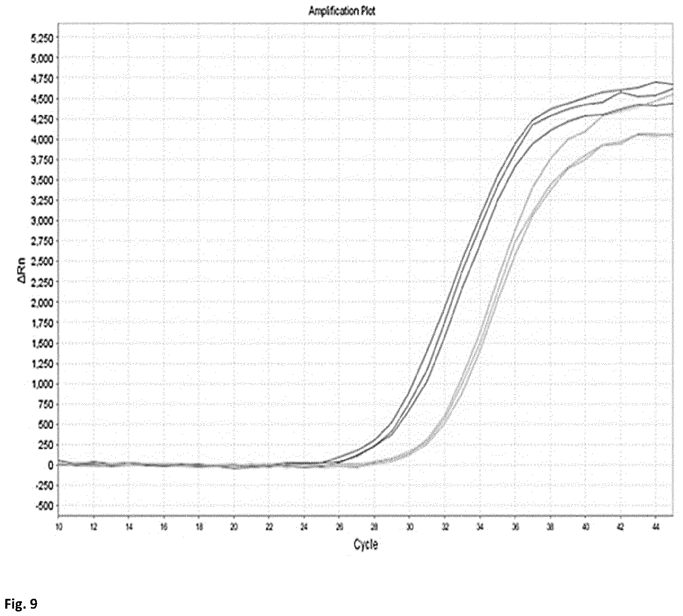

The table below, as well as figure 9, show comparative results of the PCR vs ONE STEP RT-qPCR for amplification of human RNAse-P template from a clinical blood sample detected using the UNIVERSAL probe (which anneals with the tagged sequence attached to the forward primer):

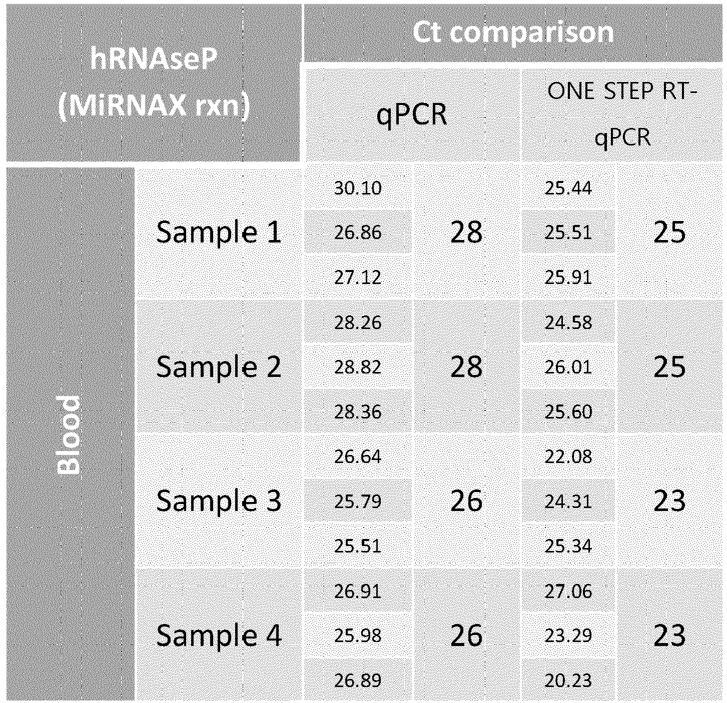

Comparative results of PCR (light blue) vs ONE STEP RT-qPCR (dark blue) for amplification of human RNAse-P template from a clinical blood sample.

### EXAMPLE 5. ANALYSIS USING ROCHE 454 SYSTEM FOR NEXT GENERATION SEQUENCING (NGS) USING AMPLICONS OBTAINED IN ACCORDANCE WITH THE PRESENT INVENTION.

For this example, we used the following reagents for the detection of the gDNA encoding the human RNAse-P enzyme:
- RNase P Primer F (TAG sequence in bold): 5'-(**GATCGATCGATC**)AGA TTTGGACCTGCGAGCG-3'
- RNase P Primer R: 5'-GAGCGGCTGTCTCCACAAGT-3'
- UNIVERAL Probe: 5'-FAM/VIC-GATCGATCGATC-BHQ1-3'

qPCR reaction master-mix and qPCR parameters for amplification with the TaqMan® Universal PCR Master Mix (Applied Biosystems) were as follows:

– PCR Master Mix: 12.5 μl
– RnaseP Fw : 0.5 μl
– RnaseP Rv: 0.5 μl
– RnaseP Probe : 0.5 μl
– H2O: 6.0 μl
Mixture volume: 20 μl

Initial denaturation 95 °C 10 min

Denaturation 95 °C 15 sec
Annealing 60 °C 60 sec
45 cycles

The resulting amplicons were assessed through random enzymatic of the PCR products sequenced via NGS. This method produced a good-quality sequencing library for the 454 platform used to test the single nucleotide polymorphisms (SNPs) located in chromosome 1 [Chr 1 (hg19)]. Eight overlapping fragments completed the hRNAse-P gene and were amplified from a single extract as described.

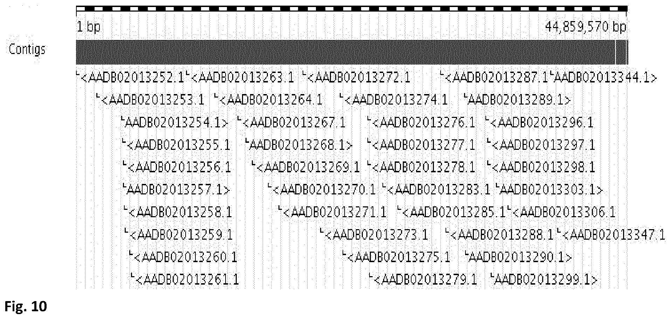

Fragments were titrated and pooled to generate the sequencing library introducing the labelled amplicons (Roche Multiplex Identifier, MID) and sequenced in Roche GS Junior device (see figure 10).

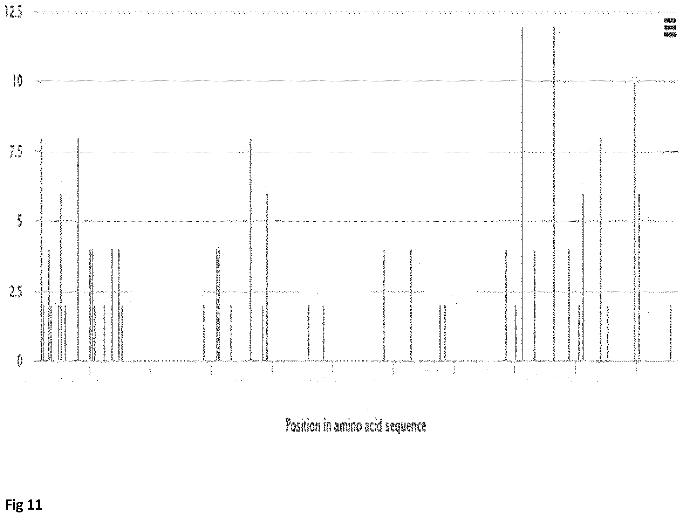

Contigs aligned to reference sequence. Lineage: Eukaryota, Metazoa, Chordata, Craniata, Vertebrata, Euteleostomi, Mammalia, Eutheria, Euarchontoglires, Primates, Haplorrhini, Catarrhini, Hominidae, Homo (see figure 11).

## Claims

1. A method for detecting or amplifying a RNA target nucleotide in a sample, preferably a human biological sample, performed in a one-step approach combining the reverse transcription and subsequent polymerase chain reaction in a single tube and buffer, wherein the method comprises:
a. carrying-out a retro-transcription reaction by using **a bivalent primer** that acts as a forward primer for the conservative step of retro-transcription of the RNA target nucleotide and forms a first strand cDNA product from the said RNA target nucleotide; and
b. carrying-out a polymerase chain reaction by using a forward primer that hybridizes with the first strand cDNA product which is then extended to form a second cDNA strand product; wherein then the bivalent primer then continues the amplification reaction over the second strand product acting as a reverse primer for the polymerase chain reaction;
wherein the bivalent primer comprises a **first sequence** of a given base length complementary to one primer portion of the RNA target nucleotide that serves directly as the template upon which the primer can anneal and to one primer portion of the second strand or cDNA product that serves directly as the template upon which the primer can anneal; and a second sequence of a given base length provided adjacent to the side of 3'terminus of said first sequence which is non-complementary with the cDNA products and **allows end-tagging the amplicon(s) obtained.**

2. The method of claim 1, wherein the RNA target nucleotide is between 18 to 25 nucleotides in length, wherein the method is **characterized in that** the DNA polymerase used for carrying-out the polymerase chain reaction possesses a 3'→5' exonuclease activity; and wherein the method is further **characterized in that** a detection probe of from 13 to 21 nucleotides in length is used for detecting the amplified products of the PCR reaction, and wherein said probe is further **characterized in that** said probe is sufficiently complementary to the cDNA products to hybridize under the selected reaction conditions and wherein said complementarity overlaps in one, two or three nucleotides with the primer portion of the cDNA product that serves directly, or by virtue of its complement, as the template upon which the bivalent primer or the forward primer anneal.

3. The method of claim 2, wherein the RNA target nucleotide is a miRNA

4. A method for isolating or purifying the amplified products of a RNA target nucleotide, preferably a miRNA, of between 18 to 25 nucleotides in length, in a sample, preferably a human biological sample, performed in a one-step approach combining the reverse transcription and subsequent polymerase chain reaction in a single tube and buffer, wherein the method comprises:
a. carrying-out a retro-transcription reaction by using a bivalent primer that acts as a forward primer for the conservative step of retro-transcription of the RNA target nucleotide and forms a first strand cDNA product from the said RNA target nucleotide; and
b. carrying-out a polymerase chain reaction by using a forward primer that hybridizes with the first strand cDNA product which is then extended to form a second cDNA strand product; wherein then the bivalent primer then continues the amplification reaction over the second strand product acting as a reverse primer for the polymerase chain reaction; and
c. isolating or purifying the amplified products;
**characterized in that** the DNA polymerase used for carrying-out the polymerase chain reaction possesses a 3'→5' exonuclease activity; and preferably **characterized in that** a detection probe of from 13 to 21 nucleotides in length is used for detecting the amplified products of the PCR reaction, and further **characterized in that** said probe is sufficiently complementary to the cDNA products to hybridize under the selected reaction conditions, wherein said complementarity overlaps in one, two or three nucleotides with the primer portion of the cDNA product that serves directly, or by virtue of its complement, as the template upon which the bivalent primer or the forward primer anneal.

5. A method for isolating or purifying the amplified products resultant from the method of claim 1, which comprises isolating or purifying the amplified products **by using the end-tag sequences of the amplicon(s) obtained.**

6. The method of any of claims 2 to 4, wherein said reverse primer is further **characterized in that** the second sequence is between 9 and 31, preferably between 16 to 24, nucleotides long, and wherein the first sequence is between 4 and 8, preferably between 4 and 6, nucleotides in length.

7. The method of any of claims 1 to 6, wherein the forward primer has a structure comprising **a first sequence** of a given base length complementary to one primer portion of the first cDNA product that serves directly as the template upon which the primer can anneal; and a second sequence of a given base length provided upstream of said first sequence which is non-complementary with the cDNA products and **allows end-tagging the amplicon(s) obtained.**

8. The method of claim 7, wherein said forward primer is further **characterized in that** the second sequence is between 9 and 31, preferably between 16 to 24, nucleotides long, and wherein the first sequence is between 4 and 8, preferably between 4 and 6, nucleotides in length.

9. The method of any of claims 2 to 4 and 6 to 8, wherein the detection probes are from about 17 to about 21 nucleotides in length.

10. The method of claim 1 to 3 and 6 to 9, wherein the method further analyses the results of the amplified products, and preferably determines the presence or absence of the RNA target nucleotide in the biological sample.

11. Amplified products obtained or obtainable by the method of any of claims 4 or 5.

12. A kit for use in preparing and/or identifying amplified amounts of DNA from a template RNA(s), the kit **characterized by** at least comprising a set of PCR primers, wherein at least one of such primers is the bivalent reverse primer as described in the precedent claims.

13. The kit according to claim 12, wherein the kit is **characterized by** at least comprising a set of PCR primers, wherein at least one of such primers is the bivalent reverse primer as described in the precedent claims and the detection probes as described in claim 2.

14. The kit according to claim 12, wherein the kit is **characterized by** at least comprising a set of PCR primers, wherein at least one of such primers is the bivalent reverse primer and the forward primer as described in the precedent claims and optionally the detection probes as described in claim 2.

15. The kit according to claim 12, wherein the kit is **characterized by** at least comprising a set of PCR primers, wherein at least one of such primers is the bivalent reverse primer and the forward primer as described in the precedent claims and the detection probes as described in claim 2, as well as a DNA polymerase used for carrying-out the polymerase chain reaction which possesses a 3'→5' exonuclease activity and/or a reverse transcriptase.

16. The kit according to claim 12, wherein the kit is **characterized by** at least comprising a set of PCR primers, wherein at least one of such primers is the bivalent reverse primer and the forward primer as described in the precedent claims and the detection probes as described in the precedent claims, as well as a DNA polymerase used for carrying-out the polymerase chain reaction which possesses a 3'→5' exonuclease activity and a reverse transcriptase, at least one of dNTPs and a buffer composition.

17. In vitro use of the kit as defined in any of claims 12 to 16, to implement the methodology of any of claims 1 to 11.
